(19) Europäisches Patentamt
European Patent Office
Office européen des brevets

(11) **EP 1 621 628 B1**

(12) **EUROPEAN PATENT SPECIFICATION**

(45) Date of publication and mention
of the grant of the patent:
**08.06.2011 Bulletin 2011/23**

(51) Int Cl.:
*C12N 9/42* (2006.01)    *A61K 38/47* (2006.01)
*A61P 3/06* (2006.01)    *A23K 1/165* (2006.01)
*C12P 19/14* (2006.01)   *A01H 5/00* (2006.01)

(21) Application number: **05018125.4**

(22) Date of filing: **20.03.2001**

(54) **Talaromyces emersonii beta-glucanases**

Talaromyces emersonii Beta-Glukanasen

Talaromyces emersonii beta-glucanases

(84) Designated Contracting States:
**AT BE CH CY DE DK ES FI FR GB GR IE IT LI LU MC NL PT SE TR**

(30) Priority: **20.03.2000 EP 00302263**

(43) Date of publication of application:
**01.02.2006 Bulletin 2006/05**

(62) Document number(s) of the earlier application(s) in accordance with Art. 76 EPC:
**01936109.6 / 1 272 643**

(73) Proprietor: **BASF SE**
**67056 Ludwigshafen (DE)**

(72) Inventors:
- **Van den Homberg, Johan.**
  **3454 AR De Meern (NL)**
- **Van der Laan, Jan-Metzke**
  **4839 AP Breda (NL)**
- **Daran, Jean-Marc Georges**
  **59800 Lille (FR)**
- **Teufel, Daniel Paul**
  **88145 Hergatz (DE)**

(56) References cited:
**US-A- 3 880 742**

- **PENTTILA M ET AL: "HOMOLOGY BETWEEN CELLULASE GENES OF TRICHODERMA-REESEI COMPLETE NUCLEOTIDE SEQUENCE OF THE ENDOGLUCANASE I GENE" GENE (AMSTERDAM), vol. 45, no. 3, 1986, pages 253-263, XP002360845 ISSN: 0378-1119**
- **MOLONEY A P ET AL: "ISOLATION AND CHARACTERIZATION OF THE 1 4-BETA-D GLUCAN GLUCANOHYDROLASES EC-3.2.1.4 OF TALAROMYCES-EMERSONII" BIOCHEMICAL JOURNAL, vol. 225, no. 2, 1985, pages 365-374, XP000929303 ISSN: 0264-6021**
- **KITAMOTO N ET AL: "Molecular cloning, purification and characterization of two endo-1,4-beta-glucanases from Aspergillus oryzae KBN616" APPLIED MICROBIOLOGY AND BIOTECHNOLOGY, SPRINGER VERLAG, BERLIN, DE, vol. 46, no. 5/6, 1996, pages 538-544, XP002104210 ISSN: 0175-7598**
- **MORRISON J ET AL: "COMPLEMENTARY DNA CLONING AND EXPRESSION OF A TALAROMYCES-EMERSONII BETA GLUCOSIDASE DETERMINANT IN ESCHERICHIA-COLI" BIOCHIMICA ET BIOPHYSICA ACTA, vol. 1049, no. 1, 1990, pages 27-32, XP000929416 ISSN: 0006-3002**
- **MURRAY, P. G. ET AL: "Isolation and characterization of a thermostable endo-.beta.-glucanase active on 1,3-1,4-.beta.-d-glucans from the aerobic fungus talaromyces emersonii CBS 814.70" ENZYME MICROB. TECHNOL., vol. 29, no. 1, 2001, pages 90-98, XP001015796**

EP 1 621 628 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## SEQUENCE LISTING

<110> DSM NV

<120> NOVEL BETA GLUCANASE

<130> N78454A

<140>
<141>

<150> GB 00302263.9
<151> 2000-03-20

<160> 8

<170> PatentIn version 3.0

<210> 1.
<211> 1008
<212> DNA
<213> Talaromyces emersonii

<220>
<221> CDS
<222> (1)..(1008)

<400> 1

```
atg aag ttc agc agg gtc gtg tgc ggt ctg acg gcc gca ggg ggc gcc    48
Met Lys Phe Ser Arg Val Val Cys Gly Leu Thr Ala Ala Gly Gly Ala
 1               5                  10                      15

ctc gcc gct cca gtc aag gag aag ggc atc aag aag cgg gcg tct ccg    96
Leu Ala Ala Pro Val Lys Glu Lys Gly Ile Lys Lys Arg Ala Ser Pro
                20                  25                  30

ttt caa tgg ttc gga tcc aac gag tct ggc gca gag ttt ggg aac aac   144
Phe Gln Trp Phe Gly Ser Asn Glu Ser Gly Ala Glu Phe Gly Asn Asn
            35                  40                  45

aac atc cct ggc gtg gag ggc acc gac tac acc ttc ccc aac acg agc   192
Asn Ile Pro Gly Val Glu Gly Thr Asp Tyr Thr Phe Pro Asn Thr Ser
        50                  55                  60

gcc atc cag atc ctc atc gac cag ggc atg aac atc ttc cgc gtg ccg   240
Ala Ile Gln Ile Leu Ile Asp Gln Gly Met Asn Ile Phe Arg Val Pro
    65                  70                  75                  80

ttc ctg atg gag cgc atg gtg ccc aac cag atg acg ggg ccg gtg gat   288
Phe Leu Met Glu Arg Met Val Pro Asn Gln Met Thr Gly Pro Val Asp
                85                  90                  95
```

```
tcg gcg tat ttc cag ggc tac agc cag gtt atc aac tac att acc agc    336
Ser Ala Tyr Phe Gln Gly Tyr Ser Gln Val Ile Asn Tyr Ile Thr Ser
            100             105             110

cat ggc gcg tcg gca gtg att gac ccg cat aac ttc ggg cga tac tac    384
His Gly Ala Ser Ala Val Ile Asp Pro His Asn Phe Gly Arg Tyr Tyr
        115             120             125

aac aat atc atc tcc tcg ccg tct gac ttc cag act ttc tgg cac act    432
Asn Asn Ile Ile Ser Ser Pro Ser Asp Phe Gln Thr Phe Trp His Thr
    130             135             140

att gcg tcc aac ttt gcg gat aat gac aat gtc att ttc gac acg aac    480
Ile Ala Ser Asn Phe Ala Asp Asn Asp Asn Val Ile Phe Asp Thr Asn
145             150             155             160

aac gaa tac cac gac atg gac gaa agc ctt gtc gtc cag ctc aac cag    528
Asn Glu Tyr His Asp Met Asp Glu Ser Leu Val Val Gln Leu Asn Gln
            165             170             175

gcc gcc atc gac ggc atc cgc gcc gcg ggc gcc aca tca cag tac atc    576
Ala Ala Ile Asp Gly Ile Arg Ala Ala Gly Ala Thr Ser Gln Tyr Ile
        180             185             190

ttc gtc gag ggc aac tcg tgg acc ggg gcc tgg aca tgg acg cag gtc    624
Phe Val Glu Gly Asn Ser Trp Thr Gly Ala Trp Thr Trp Thr Gln Val
        195             200             205

aac gac gcg atg gcg aac ctg acg gac ccg cag aac aag atc gtg tac    672
Asn Asp Ala Met Ala Asn Leu Thr Asp Pro Gln Asn Lys Ile Val Tyr
210             215             220

gag atg cac cag tac ctg gac tcg gac ggg tcg ggc acg tcg gac cag    720
Glu Met His Gln Tyr Leu Asp Ser Asp Gly Ser Gly Thr Ser Asp Gln
225             230             235             240

tgc gtc aac tcg acc atc ggg cag gac cgc gtc gag tcg gcg acg gcc    768
Cys Val Asn Ser Thr Ile Gly Gln Asp Arg Val Glu Ser Ala Thr Ala
            245             250             255

tgg ctg aag cag aac ggc aag aag gcg atc ctg ggc gag tac gct ggc    816
Trp Leu Lys Gln Asn Gly Lys Lys Ala Ile Leu Gly Glu Tyr Ala Gly
            260             265             270

ggc gcc aac agc gtg tgc gag acg gcc gtc acc ggc atg ctc gac tat    864
Gly Ala Asn Ser Val Cys Glu Thr Ala Val Thr Gly Met Leu Asp Tyr
        275             280             285

ctc gcc aac aat act gat gtc tgg acc ggt gct atc tgg tgg gcg gct    912
Leu Ala Asn Asn Thr Asp Val Trp Thr Gly Ala Ile Trp Trp Ala Ala
        290             295             300

ggg ccg tgg tgg gga gac tat atc ttc tcc atg gag ccg cct agt ggg    960
Gly Pro Trp Trp Gly Asp Tyr Ile Phe Ser Met Glu Pro Pro Ser Gly
305             310             315             320

att gcg tat gag cag gtt ctg ccg ttg ctg aag ccg tac ctc gaa tga   1008
Ile Ala Tyr Glu Gln Val Leu Pro Leu Leu Lys Pro Tyr Leu Glu ***
            325             330             335
```

<210> 2
<211> 335
<212> PRT
<213> Talaromyces emersonii

<400> 2

```
Met Lys Phe Ser Arg Val Val Cys Gly Leu Thr Ala Ala Gly Gly Ala
 1               5                   10                  15

Leu Ala Ala Pro Val Lys Glu Lys Gly Ile Lys Lys Arg Ala Ser Pro
            20                  25                  30

Phe Gln Trp Phe Gly Ser Asn Glu Ser Gly Ala Glu Phe Gly Asn Asn
        35                  40                  45

Asn Ile Pro Gly Val Glu Gly Thr Asp Tyr Thr Phe Pro Asn Thr Ser
    50                  55                  60

Ala Ile Gln Ile Leu Ile Asp Gln Gly Met Asn Ile Phe Arg Val Pro
65                  70                  75                  80

Phe Leu Met Glu Arg Met Val Pro Asn Gln Met Thr Gly Pro Val Asp
            85                  90                  95

Ser Ala Tyr Phe Gln Gly Tyr Ser Gln Val Ile Asn Tyr Ile Thr Ser
        100                 105                 110

His Gly Ala Ser Ala Val Ile Asp Pro His Asn Phe Gly Arg Tyr Tyr
        115                 120                 125

Asn Asn Ile Ile Ser Ser Pro Ser Asp Phe Gln Thr Phe Trp His Thr
    130                 135                 140

Ile Ala Ser Asn Phe Ala Asp Asn Asp Asn Val Ile Phe Asp Thr Asn
145                 150                 155                 160

Asn Glu Tyr His Asp Met Asp Glu Ser Leu Val Val Gln Leu Asn Gln
            165                 170                 175

Ala Ala Ile Asp Gly Ile Arg Ala Ala Gly Ala Thr Ser Gln Tyr Ile
            180                 185                 190

Phe Val Glu Gly Asn Ser Trp Thr Gly Ala Trp Thr Trp Thr Gln Val
    195                 200                 205

Asn Asp Ala Met Ala Asn Leu Thr Asp Pro Gln Asn Lys Ile Val Tyr
    210                 215                 220

Glu Met His Gln Tyr Leu Asp Ser Asp Gly Ser Gly Thr Ser Asp Gln
225                 230                 235                 240

Cys Val Asn Ser Thr Ile Gly Gln Asp Arg Val Glu Ser Ala Thr Ala
            245                 250                 255

Trp Leu Lys Gln Asn Gly Lys Lys Ala Ile Leu Gly Glu Tyr Ala Gly
            260                 265                 270

Gly Ala Asn Ser Val Cys Glu Thr Ala Val Thr Gly Met Leu Asp Tyr
        275                 280                 285

Leu Ala Asn Asn Thr Asp Val Trp Thr Gly Ala Ile Trp Trp Ala Ala
    290                 295                 300

Gly Pro Trp Trp Gly Asp Tyr Ile Phe Ser Met Glu Pro Pro Ser Gly
305                 310                 315                 320

Ile Ala Tyr Glu Gln Val Leu Pro Leu Leu Lys Pro Tyr Leu Glu
        325                 330                 335
```

<210> 3

<211> 1245

<212> DNA

<213> Talaromyces emersonii

<220>
<221> CDS
<222> (1)..(1245)

<400> 3

```
atg gat cga att ctt gcg ctg atc ttg gtc ccc ctt gcc act gtc acg    48
Met Asp Arg Ile Leu Ala Leu Ile Leu Val Pro Leu Ala Thr Val Thr
1               5                   10                  15

gca cag cag att ggc act atc ccc gag gtc cat ccc aag ctc ccg aca    96
Ala Gln Gln Ile Gly Thr Ile Pro Glu Val His Pro Lys Leu Pro Thr
                20                  25                  30

tgg aaa tgc acg acc gag ggc ggc tgt gtc cag cag aat acc tcc gtc   144
Trp Lys Cys Thr Thr Glu Gly Gly Cys Val Gln Gln Asn Thr Ser Val
            35                  40                  45

gtg ttg gag tac ctg tcg cat ccg atc cat gaa gtt gga aac agc gac   192
Val Leu Glu Tyr Leu Ser His Pro Ile His Glu Val Gly Asn Ser Asp
        50                  55                  60

gtc tcg tgc gtg gtt tct ggc ggg ctg aac cag agc ctc tgt ccc aac   240
Val Ser Cys Val Val Ser Gly Gly Leu Asn Gln Ser Leu Cys Pro Asn
65                  70                  75                  80

gaa gag gaa tgt tcc aaa aac tgc gtc gtc gag ggg gcc aac tac acc   288
Glu Glu Glu Cys Ser Lys Asn Cys Val Val Glu Gly Ala Asn Tyr Thr
                85                  90                  95

agc tcg gga gtt cac aca gac ggc gat gcc ctg act ctc aat cag tac   336
Ser Ser Gly Val His Thr Asp Gly Asp Ala Leu Thr Leu Asn Gln Tyr
                100                 105                 110

gtc acg aac ggc gac cag gtc gtc acc gcc tcg ccg cgg gtc tat ctc   384
Val Thr Asn Gly Asp Gln Val Val Thr Ala Ser Pro Arg Val Tyr Leu
            115                 120                 125

ctg gcc agc gac gac gag gac ggg aat tac agc atg ctc cag ctc ctc   432
Leu Ala Ser Asp Asp Glu Asp Gly Asn Tyr Ser Met Leu Gln Leu Leu
        130                 135                 140

ggc cag gag ctg agc ttt gac gtg gac gtc tcg aaa ctg gtc tgc ggg   480
Gly Gln Glu Leu Ser Phe Asp Val Asp Val Ser Lys Leu Val Cys Gly
145                 150                 155                 160

atg aac ggc gcc ttg tat ctc tcc gag atg gac gca tcg ggc ggc cga   528
Met Asn Gly Ala Leu Tyr Leu Ser Glu Met Asp Ala Ser Gly Gly Arg
                165                 170                 175

aac agc ctc aac ccg gcg ggg gca cag tat ggc tct gga tac tgt gat   576
Asn Ser Leu Asn Pro Ala Gly Ala Gln Tyr Gly Ser Gly Tyr Cys Asp
                180                 185                 190
```

```
gcg caa tgc ggc gtc cag ccc ttc atc aac ggc acg gtc aac acc ggc      624
Ala Gln Cys Gly Val Gln Pro Phe Ile Asn Gly Thr Val Asn Thr Gly
        195                 200                 205

tcg ctc ggc gct tgc tgc aac gag atg gac atc tgg gaa gcg aat gcc      672
Ser Leu Gly Ala Cys Cys Asn Glu Met Asp Ile Trp Glu Ala Asn Ala
        210                 215                 220

ctt gcc acc gcg ttg act ccg cac ccg tgc agc gtc acc agc atc tat      720
Leu Ala Thr Ala Leu Thr Pro His Pro Cys Ser Val Thr Ser Ile Tyr
225                 230                 235                 240

gcc tgt tct ggc gct gag tgc ggc tcc aac ggc gtc tgc gac aag ccg      768
Ala Cys Ser Gly Ala Glu Cys Gly Ser Asn Gly Val Cys Asp Lys Pro
                245                 250                 255

gga tgc gga tac aac ccg tac gcg ctg gga gac cac aac tac tac gga      816
Gly Cys Gly Tyr Asn Pro Tyr Ala Leu Gly Asp His Asn Tyr Tyr Gly
                260                 265                 270

ccc ggg aag acg gtc gac acg tcc agg ccc ttc acc gtg gta acg cag      864
Pro Gly Lys Thr Val Asp Thr Ser Arg Pro Phe Thr Val Val Thr Gln
        275                 280                 285

ttt ctc acc aac gac aac acc acg acg ggg act ctg acg gag atc cgt      912
Phe Leu Thr Asn Asp Asn Thr Thr Thr Gly Thr Leu Thr Glu Ile Arg
        290                 295                 300

cgt ctg tac gtc caa gac ggc aac gtg atc ggg cct tcg cct agc gac      960
Arg Leu Tyr Val Gln Asp Gly Asn Val Ile Gly Pro Ser Pro Ser Asp
305                 310                 315                 320

tct gtc tcg tca atc acg gac tcg ttc tgt tcc acg gtg gat tcc tat     1008
Ser Val Ser Ser Ile Thr Asp Ser Phe Cys Ser Thr Val Asp Ser Tyr
                325                 330                 335

ttc gag ccg ctt ggc ggc ctg aag gag atg ggc gag gcg ctg ggt cgg     1056
Phe Glu Pro Leu Gly Gly Leu Lys Glu Met Gly Glu Ala Leu Gly Arg
                340                 345                 350

ggg atg gtg ctg gtg ttc agc atc tgg aat gat cct ggt cag ttc atg     1104
Gly Met Val Leu Val Phe Ser Ile Trp Asn Asp Pro Gly Gln Phe Met
                355                 360                 365

aac tgg ctc gac agc ggg aat gct ggg ccc tgc aac agc acc gag ggg     1152
Asn Trp Leu Asp Ser Gly Asn Ala Gly Pro Cys Asn Ser Thr Glu Gly
        370                 375                 380

aac cca gcg act att gaa gcg cag cat cct gac acc gcg gtg acc ttc     1200
Asn Pro Ala Thr Ile Glu Ala Gln His Pro Asp Thr Ala Val Thr Phe
385                 390                 395                 400

tcg aac atc aga tgg ggg gat atc ggg tcg acg ttc cag tcg taa        1245
Ser Asn Ile Arg Trp Gly Asp Ile Gly Ser Thr Phe Gln Ser
                405                 410
```

<210> 4

<211> 414

<212> PRT

<213> Talaromyces emersonii

<400> 4

```
Met Asp Arg Ile Leu Ala Leu Ile Leu Val Pro Leu Ala Thr Val Thr
1             5                 10                15

Ala Gln Gln Ile Gly Thr Ile Pro Glu Val His Pro Lys Leu Pro Thr
            20              25              30

Trp Lys Cys Thr Thr Glu Gly Gly Cys Val Gln Gln Asn Thr Ser Val
        35          40                  45

Val Leu Glu Tyr Leu Ser His Pro Ile His Glu Val Gly Asn Ser Asp
    50              55              60

Val Ser Cys Val Val Ser Gly Gly Leu Asn Gln Ser Leu Cys Pro Asn
65              70                  75                  80

Glu Glu Glu Cys Ser Lys Asn Cys Val Val Glu Gly Ala Asn Tyr Thr
            85                  90                  95

Ser Ser Gly Val His Thr Asp Gly Asp Ala Leu Thr Leu Asn Gln Tyr
        100             105             110

Val Thr Asn Gly Asp Gln Val Val Thr Ala Ser Pro Arg Val Tyr Leu
    115                 120             125

Leu Ala Ser Asp Asp Glu Asp Gly Asn Tyr Ser Met Leu Gln Leu Leu
    130             135             140

Gly Gln Glu Leu Ser Phe Asp Val Asp Val Ser Lys Leu Val Cys Gly
145             150                 155                 160

Met Asn Gly Ala Leu Tyr Leu Ser Glu Met Asp Ala Ser Gly Gly Arg
            165             170             175

Asn Ser Leu Asn Pro Ala Gly Ala Gln Tyr Gly Ser Gly Tyr Cys Asp
            180             185             190

Ala Gln Cys Gly Val Gln Pro Phe Ile Asn Gly Thr Val Asn Thr Gly
        195             200             205

Ser Leu Gly Ala Cys Cys Asn Glu Met Asp Ile Trp Glu Ala Asn Ala
    210             215             220

Leu Ala Thr Ala Leu Thr Pro His Pro Cys Ser Val Thr Ser Ile Tyr
225             230             235             240

Ala Cys Ser Gly Ala Glu Cys Gly Ser Asn Gly Val Cys Asp Lys Pro
            245             250             255

Gly Cys Gly Tyr Asn Pro Tyr Ala Leu Gly Asp His Asn Tyr Tyr Gly
            260             265             270

Pro Gly Lys Thr Val Asp Thr Ser Arg Pro Phe Thr Val Val Thr Gln
        275             280             285

Phe Leu Thr Asn Asp Asn Thr Thr Thr Gly Thr Leu Thr Glu Ile Arg
    290             295             300

Arg Leu Tyr Val Gln Asp Gly Asn Val Ile Gly Pro Ser Pro Ser Asp
305             310             315             320

Ser Val Ser Ser Ile Thr Asp Ser Phe Cys Ser Thr Val Asp Ser Tyr
            325             330             335
```

38

```
Phe Glu Pro Leu Gly Gly Leu Lys Glu Met Gly Glu Ala Leu Gly Arg
            340             345             350

Gly Met Val Leu Val Phe Ser Ile Trp Asn Asp Pro Gly Gln Phe Met
        355             360             365

Asn Trp Leu Asp Ser Gly Asn Ala Gly Pro Cys Asn Ser Thr Glu Gly
    370             375             380

Asn Pro Ala Thr Ile Glu Ala Gln His Pro Asp Thr Ala Val Thr Phe
385             390             395             400

Ser Asn Ile Arg Trp Gly Asp Ile Gly Ser Thr Phe Gln Ser
            405             410
```

<210> 5
<211> 669
<212> DNA
<213> Talaromyces emersonii

<220>
<221> CDS
<222> (1)..(669)

<400> 5

```
atg aat gtc aga gct gtt gtc tct gtt tct gcc ttc ttg ctg acg cct      48
Met Asn Val Arg Ala Val Val Ser Val Ser Ala Phe Leu Leu Thr Pro
1               5               10              15

ttg gct tca gcc ctg aca gga acc acc aca aca aca tgg gac tgt tgt      96
Leu Ala Ser Ala Leu Thr Gly Thr Thr Thr Thr Thr Trp Asp Cys Cys
            20              25              30

aaa cca gcg tgt agc tgg acg caa aat gcc caa gca ggc gga gca agt     144
Lys Pro Ala Cys Ser Trp Thr Gln Asn Ala Gln Ala Gly Gly Ala Ser
        35              40              45

ggt acc gtc gcc acc tgc aac atc aac aac cag gta ctc agc aat ggt     192
Gly Thr Val Ala Thr Cys Asn Ile Asn Asn Gln Val Leu Ser Asn Gly
    50              55              60

gcc tct gct ccc agc gcc tgc cag gga ggc gat gcc tac agc tgc tcc     240
Ala Ser Ala Pro Ser Ala Cys Gln Gly Gly Asp Ala Tyr Ser Cys Ser
65              70              75              80

gac ttc cag ccc atc atc atc agt gac acg ttg tcg tac gga ttc gct     288
Asp Phe Gln Pro Ile Ile Ile Ser Asp Thr Leu Ser Tyr Gly Phe Ala
                85              90              95

ggc aac tgg gag aca agc aac tgc tgc aag tgc ttc cag ttc acc tgg     336
Gly Asn Trp Glu Thr Ser Asn Cys Cys Lys Cys Phe Gln Phe Thr Trp
            100             105             110

acg tcg ggg gcg ggt gcg ggc aag tcc atg atc gtc caa gtt gtc aat     384
Thr Ser Gly Ala Gly Ala Gly Lys Ser Met Ile Val Gln Val Val Asn
```

39

<pre>
                   115                      120                      125

     tct ggc ggg gtc agt aca ggc gat ttc gac att tac acg cct ggt ggc        432
     Ser Gly Gly Val Ser Thr Gly Asp Phe Asp Ile Tyr Thr Pro Gly Gly
         130                     135                     140

     ggt gtt gga gat tac aat gcc tgc act tcg cag tat ggc gcg cca cca        480
     Gly Val Gly Asp Tyr Asn Ala Cys Thr Ser Gln Tyr Gly Ala Pro Pro
     145                     150                     155                 160

     cag gga tgg ggt gct caa tac ggc ggc gtc tcc agc gac gct gaa tgc        528
     Gln Gly Trp Gly Ala Gln Tyr Gly Gly Val Ser Ser Asp Ala Glu Cys
                         165                     170                     175

     gac caa ctc ccc tcg atc ctg cag cct gga tgc cac tgg cgt ttc gag        576
     Asp Gln Leu Pro Ser Ile Leu Gln Pro Gly Cys His Trp Arg Phe Glu
                     180                     185                     190

     tgg gca gga ggt ggc atc aac gga tgg acg act gag tac gag gaa gtc        624
     Trp Ala Gly Gly Gly Ile Asn Gly Trp Thr Thr Glu Tyr Glu Glu Val
                 195                     200                     205

     gat tgc cca agc cag ctt act tcc atc tca ggt tgc tat ccg tga        669
     Asp Cys Pro Ser Gln Leu Thr Ser Ile Ser Gly Cys Tyr Pro
         210                     215                     220
</pre>

<210> 6

<211> 222

<212> PRT

<213> Talaromyces emersonii

<400> 6

<pre>
     Met Asn Val Arg Ala Val Val Ser Val Ser Ala Phe Leu Leu Thr Pro
     1               5               10                      15

     Leu Ala Ser Ala Leu Thr Gly Thr Thr Thr Thr Thr Trp Asp Cys Cys
                 20                  25                  30

     Lys Pro Ala Cys Ser Trp Thr Gln Asn Ala Gln Ala Gly Gly Ala Ser
             35                  40                  45

     Gly Thr Val Ala Thr Cys Asn Ile Asn Asn Gln Val Leu Ser Asn Gly
         50                  55                  60

     Ala Ser Ala Pro Ser Ala Cys Gln Gly Gly Asp Ala Tyr Ser Cys Ser
     65                  70                  75                  80

     Asp Phe Gln Pro Ile Ile Ile Ser Asp Thr Leu Ser Tyr Gly Phe Ala
                     85                  90                  95

     Gly Asn Trp Glu Thr Ser Asn Cys Cys Lys Cys Phe Gln Phe Thr Trp
                 100                 105                 110

     Thr Ser Gly Ala Gly Ala Gly Lys Ser Met Ile Val Gln Val Val Asn
                 115                 120                 125

     Ser Gly Gly Val Ser Thr Gly Asp Phe Asp Ile Tyr Thr Pro Gly Gly
         130                 135                 140
</pre>

```
Gly Val Gly Asp Tyr Asn Ala Cys Thr Ser Gln Tyr Gly Ala Pro Pro
145              150              155              160

Gln Gly Trp Gly Ala Gln Tyr Gly Gly Val Ser Ser Asp Ala Glu Cys
              165              170              175

Asp Gln Leu Pro Ser Ile Leu Gln Pro Gly Cys His Trp Arg Phe Glu
            180              185              190

Trp Ala Gly Gly Gly Ile Asn Gly Trp Thr Thr Glu Tyr Glu Glu Val
            195              200              205

Asp Cys Pro Ser Gln Leu Thr Ser Ile Ser Gly Cys Tyr Pro
    210              215              220
```

<210> 7
<211> 28
<212> DNA
<213> Artificial Sequence

<220>
<223> oligonucleotide primer

<400> 7
tatagcgaaa tggattgatt gtacgctc

<210> 8
<211> 26
<212> DNA
<213> Artificial Sequence

<220>
<223> oligonucleotide primer

<400> 8
atccccagca tcattacacc tcagtg

**Description**

Field of Invention

The present invention relates to novel (β-)glucanases (and so also to cellulases) from the fungus *Talaromyces*, in particular *Talaromyces emersonii*, and their use in degrading glucan in cellulose.

Background of the invention

The composition of a plant cell wall-is complex and variable. Polysaccharides are mainly found in the form of long chains of cellulose (the main structural component of the plant cell wall), hemicellulose (comprising various β-xylan chains, such as xyloglucans) and pectin.

Beta-glucan (or, more properly, (1→3)(1→4)β-D-glucan), MW 10 to 14 kDa, is a component of plant hemicellulose (MW of about 700 kDa) and consists of a backbone of β-1,4 and 1,3-linked glucopyranose residues. Another component is xylan which consists of β-1,4-linked xylose residues, optionally substituted with side chains such as arabinose and/or glucuronic acid residues.

Basic differences exist between monocotyledons (e.g. cereals and grasses) and dicotyledons (e.g. clover, rapeseed and soybean) and between the seed and vegetative parts of the plant Monocotyledons are characterized by the presence of an arabinoxylan complex as the major hemicellulose backbone, and the main structure of hemicellulose in dicotyledons is a xyloglucan complex. Higher pectin concentrations are found in dicotyledons than in monocotyledons. Seeds are generally high in pectic substances but relatively low in cellulosic material.

Cellulose degrading enzymes are used for the processing of plant material in food as well as feed applications or as a food or feed additive due to of their capability to act on main plant cell wall substituents.

Most of the cellulose degrading enzymes available to the industry appear to be glucanases with a relatively low molecular weight and a moderate stability at higher temperatures.

MOLONEY A. P. et al. (BIOCHEMICAL JOURNAL, Vol. 225, No. 2, 1985, pages 365-374) discloses four endo-Glucanases from T. emersonii that show an optimal activity at a pH from 5,5 to 5,8 and at a temperature from 75 °C to 80 °C.

However, for certain applications it is desirable to use a glucanase with a relatively high thermostability. If a glucanase is to be used as an animal feed additive then a high thermostability is preferred because of the high temperature conditions applied during pelleting the animal feed.

Summary of the Invention

Novel β-glucanases (or cellulases) are now provided which are able to cleave β-D-glucan (or cellulose) such as present in plant material.

There is provided a polypeptide, which is a β-glucanase obtainable from a fungus of the genus Talaromyces, such as the fungus Talaromyces emersonii, having the activity EC 3.2.1.4 (endoglucanase activity), comprising:

  (i) the amino acid sequence of SEQ ID No. 4; or
  (ii) a variant of (i) which is capable of cleaving β-D-glucan having at least 80 % identity over the whole length to the amino acid sequence of SEQ ID No. 4; or (iii) a fragment of (i) or (ii) which is capable of cleaving β-D-glucan.

At its broadest, the invention relates to β-glucanases (or cellulases) from *Talaromyces,* such as *Talaromyces emersonii* (a fungus). These β-glucanases can cleave β-D-glucan or cellulose, for example they are β-1,4-endoglucanases, or have the activity EC 3.2.1.4.

The glucanases may have:

  a. an optimum pH below 7.0 or 5.4, such as below 5.0, for example from 4.4 to 5.2 or from 3.0 to 6.0 or 7.0; or
  b. a temperature optimum of at least 72°C, 75°C or even 81°C, such as from 78°C to 85°C or from 83°C to 87°C.

More specifically, the present invention provides an (isolated) β-glucanase polypeptide comprising:

  the amino acid sequence of SEQ ID No 4, or
  (ii) a variant of (i) which is capable of cleaving β-D-glucan; or
  (iii) a fragment of (i) or (ii) which is capable of cleaving β-D-glucan

According to another aspect of the invention there is provided a polynucleotide which comprises:

(a) the nucleic acid sequence of SEQ ID No. 3 or a sequence encoding a polypeptide according to any preceding claim;
(b) a sequence which is complementary to, or which hybridises to, a sequence as defined in (a) under stringent conditions;
(c) a fragment of at least 25 bp of SEQ ID No. 3;
(d) a sequence having at least 80 % identity to a sequence as defined in (a) or (b) ; or
(e) a sequence that is degenerate as a result of the genetic code to any one of the sequences as defined in (a) to (d)
(f) a modified sequence of Seq ID No. 3 with up to 100 nucleotide substitutions.

The invention also provides:

- an (eg. expression) vector which comprises a polynucleotide of the invention and which may be capable of expressing a polypeptide of the invention;
- a cell line or strain comprising a vector of the invention;
- a method of producing a polypeptide of the invention which method comprises maintaining a cell line or strain of the invention under conditions suitable for obtaining expression of the polypeptide and, if necessary, isolating the polypeptide;
- a method of degrading β-D-glucan, the method comprising contacting a material comprising β-D-glucan with a polypeptide of the invention;
- a method for identification of a compound that modulates β-glucanase activity, which method comprises contacting a polypeptide of the invention with a test compound in the presence of β-D-glucan and monitoring for or detecting any modulation of activity;
- a method of treating a subject having hyperlipemia which method comprises administering to the subject an effective amount of the polypeptide of the invention; and
- use of the polypeptide in the manufacture of a medicament for the treatment or prophylaxis of hyperlipemia.

Brief Description of the Sequences

SEQ ID No. 1 is a DNA sequence of a first β-glucanase (CEA) of the invention from *Talaromyces emersonii*;
SEQ ID No. 2 is the amino acid sequence of the first β-glucanase, CEA;
SEQ ID No. 3 is a DNA sequence of a second β-glucanase (CEB) from the same organism;
SEQ ID No. 4 is the amino acid sequence of the second β-glucanase, CEB;
SEQ ID No. 5 is a DNA sequence of a third β-glucanase (CEC) also from the same organism;
SEQ ID No. 6 is the amino acid sequence of the third β-glucanase, CEC; and
SEQ ID Nos. 7 and 8 are PCR primers that hybridize to SEQ ID No. 1 (and were used to reclone cDNA inserts during genetic analysis of positive transformants).

Detailed Description of the Invention

A. Polynucleotides.

The present invention provides a (e.g. isolated and/or purified) polynucleotide encoding polypeptides of the invention. The present invention thus provides a polynucleotide encoding a β-glucanase whose amino acid sequence is set out in SEQ ID No. 4. The present invention further provides a polynucleotide encoding a polypeptide having substantial amino acid sequence homology to the amino acid sequence set out in SEQ ID No. 4. Also included is a polynucleotide selected from:

(a) a polynucleotide comprising the nucleotide sequence set out in SEQ ID No. 3, or the complement thereof;
(b) a polynucleotide comprising a nucleotide sequence capable of (e.g. selectively) hybridising to a nucleotide sequence set out in SEQ ID No. 3, or a fragment thereof;
(c) a polynucleotide comprising a nucleotide sequence capable of (e.g. selectively) hybridising to the complement of the nucleotide sequence set out in SEQ ID No. 3, or a fragment thereof; and/or
(d) a polynucleotide comprising a polynucleotide sequence that is degenerate as a result of the genetic code to a polynucleotide defined in (a), (b) or (c).

A polynucleotide of the invention also includes a polynucleotide which:

(a) encodes a polypeptide having β-glucanase activity, which polynucleotide is:

(1) the coding sequence of SEQ ID No. 3;
(2) a sequence which hybridises selectively to the complement of sequence defined in (1); or
(3) a sequence that is degenerate as a result of the genetic code with respect to a sequence defined in (1) or (2); or

(b) is a sequence complementary to a polynucleotide defined in (a).

*Hybridisable sequences*

The term "capable of hybridizing" means that the target polynucleotide of the invention can hybridize to the nucleic acid used as a probe (for example the nucleotide sequence set out in SEQ. ID No 3, or a fragment thereof-or the complement thereof) at a level significantly above background. The invention also includes nucleotide sequences that encode for β-glucanase or variants thereof as well as nucleotide sequences which are complementary thereto. The nucleotide sequence may be RNA or DNA and thus includes genomic DNA, synthetic DNA or cDNA. Preferably the nucleotide sequence is a DNA sequence and most preferably, a cDNA sequence. Typically a polynucleotide of the invention comprises a contiguous sequence of nucleotides which is capable of hybridizing under selective conditions to the coding sequence or the complement of the coding sequence of SEQ ID No. 3, as appropriate. Such nucleotides can be synthesized according to methods well known in the art[1].

A polynucleotide of the invention can hybridize to the coding sequence or the complement of the coding sequence of SEQ ID No. 3 (as appropriate) at a level significantly above background. Background hybridization may occur, for example, because of other cDNAs present in a cDNA library. The signal level (eg. generated by the interaction between a polynucleotide of the invention and the coding sequence or complement of the coding sequence of SEQ ID No. 3) is typically at least 10 fold, preferably at least 100 fold, as intense as interactions between other polynucleotides and the coding sequence of SEQ ID No. 3.

The intensity of interaction may be measured, for example, by radiolabelling the probe, e.g. with $^{32}$P. Selective hybridization may typically be achieved using conditions of low stringency (0.3M sodium chloride and 0.03M sodium citrate at about 40°C), medium stringency (for example, 0.3M sodium chloride and 0.03M sodium citrate at about 50°C) or high stringency (for example, 0.3M sodium chloride and 0.03M sodium citrate at about 60°C). Hybridization may be carried out under any suitable conditions known in the art[1] and, as a guide, low stringency can be 2 x SSC at 55°C, medium stringency can be 0.5 to 1.0 x SSC at 60°C and high stringency can be 0.1 or 0.2 x SSC at 60°C or higher (e.g. at 68°C), all at 0.5% SDS.

*Modifications*

Polynucleotides of the invention may comprise DNA or RNA. They may be single or double stranded. They may also be polynucleotides which include within them one or more synthetic or modified nucleotides. A number of different types of modifications to polynucleotides are known in the art. These include methylphosphonate and phosphorothioate backbones and/or addition of acridine or polylysine chains at the 3' and/or 5' ends of the molecule. For the purposes of the present invention, it is to be understood that the polynucleotides described herein may be modified by any method available in the art.

It is to be understood that skilled persons may, using routine techniques, make nucleotide substitutions that do not affect the polypeptide sequence encoded by the polynucleotides of the invention to reflect the codon usage of any particular host organism, for example in which the polypeptides of the invention are to be expressed.

The coding sequence of SEQ ID No. 3 may be modified by nucleotide substitutions, for example from or up to 1, 2 or 3 to 10, 25, 50 or 100 substitutions. The polynucleotide may alternatively or additionally be modified by one or more insertions and/or deletions and/or by an extension at either or both ends. The modified polynucleotide generally encodes a polypeptide which has β-glucanase activity. Degenerate substitutions may be made and/or substitutions may be made which would result in a conservative amino acid substitution when the modified sequence is translated, for example as discussed with reference to polypeptides later.

*Homologues*

A nucleotide sequence which is capable of selectively hybridizing to (e.g. the complement of) the DNA coding sequence of SEQ ID No. 3 may have at least 50% or 60%, at least 70%, at least 80%, at least 90%, at least 95%, at least 98% or at least 99% sequence identity (or homology) to the coding sequence of SEQ ID No. 3. This may be over a region of at least 20, preferably at least 30, for instance at least 40, at least 60, more preferably at least 100 contiguous nucleotides or optimally over the full length of SEQ ID No. 3. For SEQ ID No. 3 the sequence identity is preferably at least 85%.

Any combination of the above mentioned degrees of homology and minimum sized may be used to define polynucleotides of the invention, with the more stringent combinations (i.e. higher homology over longer lengths) being preferred. Thus for example a polynucleotide which is at least 80% or 90% homologous over 25, preferably over 30 nucleotides forms one aspect of the invention, as does a polynucleotide which is at least 90% homologous over 40 nucleotides.

Homologues of polynucleotide (or protein) sequences typically have at least 70% homology, preferably at least 80, 90%, 95%, 97% or 99% homology, for example over a region of at least 15, 20, 30, 100 more contiguous nucleotides (or amino acids). The homology may calculated on the basis of amino acid identity (sometimes referred to as "hard homology").

For example the UWGCG Package provides the BESTFIT program which can be used to calculate homology (for example used on its default settings[5]). The PILEUP and BLAST algorithms can be used to calculate homology or line up sequences (such as identifying equivalent or corresponding sequences, for example on their default settings[6,7]).

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold[6,7]. These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X. from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix[8] alignments (B) of 50, expectation (E) of 10, M=5, N=4, and a comparison of both strands.

The BLAST algorithm performs a statistical analysis of the similarity between two sequences[9]. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

*Primers and Probes*

Polynucleotides of the invention include and may be used as a primer, e.g. a PCR primer, a primer for an alternative amplification reaction, a probe, or the polynucleotides may be cloned into vectors. Such primers, probes and other fragments will be at least or up to 20, for example at least 25, 30 or 40 nucleotides in length. They will typically be up to 40, 50, 60, 70, 100, 150, 200 or 300 nucleotides in length, or even up to a few nucleotides (such as 5 or 10 nucleotides) short of the coding sequence of SEQ ID No. 3.

In general, primers will be produced by synthetic means, involving a step-wise manufacture of the desired nucleic acid sequence one nucleotide at a time. Techniques for accomplishing this using automated techniques are readily available in the art. Examples of primers of the invention are set out in SEQ ID Nos 7 and 8.

Longer polynucleotides will generally be produced using recombinant means, for example using PCR (polymerase chain reaction) cloning techniques. This will involve making a pair of primers (e.g. of about 15-30 nucleotides) to a region of the β-glucanase which it is desired to clone, bringing the primers into contact with mRNA or cDNA obtained from a target (e.g yeast, bacterial, plant, prokaryotic or fungal) cell, preferably of an *Talaromyces* strain, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

Such techniques may be used to obtain all or part of the β-glucanase sequence described herein. Genomic clones corresponding to the cDNA of SEQ ID No. 3 or the β-glucanase gene containing, for example, introns and promoter regions are within the invention also and may also be obtained in an analogous manner (e.g. recombinant means, PCR, cloning techniques), starting with genomic DNA from a fungal, yeast, bacterial plant or prokaryotic cell.

The polynucleotides or primers may carry a revealing label, e.g. a radioactive or non-radioactive label. Suitable labels include radioisotopes such as $^{32}P$ or $^{35}S$, enzyme labels, or other protein labels such as biotin. Such labels may be added to polynucleotides or primers of the invention and may be detected using techniques known *per se.*

Polynucleotides, labelled or unlabelled may be used in nucleic acid-based tests for detecting or sequencing β-glucanase or a variant thereof in a (e.g. fungal) sample. Such tests for detecting generally comprise bringing a (e.g. fungal) sample (suspected of) containing DNA into contact with a probe or primer of the invention under hybridizing conditions and detecting any duplex formed between the probe and nucleic acid in the sample. Such detection may be achieved using techniques such as PCR or by immobilizing the probe on a solid support, removing nucleic acid in the sample which is not hybridized to the probe, and then detecting nucleic acid which was hybridized to the probe. Alternatively, the sample nucleic acid may be immobilized on a solid support, and the amount of probe bound to such a support can be detected.

The probes of the invention may conveniently be packaged in the form of a test kit in a suitable container. In such kits the probe may be bound to a solid support where the assay format for which the kit is designed requires such binding. The kit may also contain suitable reagents for treating the sample to be probed, hybridizing the probe to nucleic acid in the sample, control reagents, instructions, and the like.

Preferably, the polynucleotide of the invention is obtainable from the same organism as the polypeptide, such as a fungus, in particular a fungus of the genus *Talaromyces*.

The polynucleotides of the invention also include variants of the sequence of SEQ ID No. 3 which have β-glucanase activity. Variants may be formed by additions, substitutions and/or deletions and may have the ability to cleave a β-D-glucan polymer.

*Production ofpolynucleotides*

Polynucleotides which do not have 100% identity with SEQ ID No. 3 but fall within the scope of the invention can be obtained in a number of ways. Thus variants of the β-glucanase sequence described herein may be obtained for example by probing genomic DNA libraries made from a range of organisms, for example those discussed as sources of the polypeptides of the invention. In addition, other fungal, plant or prokaryotic homologues of β-glucanase may be obtained and such homologues and fragments thereof in general will be capable of hybridising to SEQ ID No. 3. Such sequences may be obtained by probing cDNA libraries or genomic DNA libraries from other species, and probing such libraries with probes comprising all or part of SEQ ID. 3 under conditions of medium to high stringency (as described earlier). Nucleic acid probes comprising all or part of SEQ ID No. 3 may be used to probe cDNA libraries from other species, such as those described as sources for the polypeptides of the invention.

Species homologues may also be obtained using degenerate PCR which will use primers designed to target sequences within the variants and homologues encoding conserved amino acid sequences. The primers can contain one or more degenerate positions and will be used at stringency conditions lower than those used for cloning sequences with single sequence primers against known sequences.

Alternatively, such polynucleotides-may be obtained by site directed mutagenesis of the β-glucanase sequences or variants thereof. This may be useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

The.invention includes double stranded polynucleotides comprising a polynucleotide of the invention and its complement.

The present invention also provides polynucleotides encoding the polypeptides of the invention described below. Since such polynucleotides will be useful as sequences for recombinant production of polypeptides of the invention, it is not necessary for them to be capable of hybridising to the sequence of SEQ ID No. 3, although this will generally be desirable. Otherwise, such polynucleotides may be labelled, used, and made as described above if desired.

B. Polypeptides.

The present invention relates to (e.g. (substantially) purified and/or isolated) β-glucanases (or cellulases) and variants thereof. The polypeptides of the invention may consist essentially of the amino acid sequence of SEQ ID No. 4 or of a variant of that sequence. Polypeptides may also be encoded by a polynucleotide of the invention as described above.

A polypeptide of the invention may be in an isolated or a substantially purified form. It will be understood that the polypeptide may be mixed with carriers or diluents which will not interfere with the intended purpose and/of function of the polypeptide and still be regarded as substantially isolated. It will generally comprise the polypeptide in a preparation in which more than 20%, e.g. more than 30%, 40%, 50%, 80%, 90%, 95% or 99%, by weight of the polypeptide in the preparation is a polypeptide of the invention. Routine methods can be employed to purify and/or synthesise the proteins according to the invention[1]. For some formulations (e.g. for non-pharmaceutical uses) the amount of polypeptide present may be small, for example from 0.01 to 10%, such as from 0.1 to 5%, or 2% or even from 0.2 to 1%.

Preferably, the polypeptide of the invention is obtainable from a microorganism which possesses a gene encoding an enzyme with β-glucanase (or cellulase) activity. More preferably the microorganism is fungal, and optimally a filamentous fungi. Preferred organisms are thus of the genus *Talaromyces*, such as of the species *Talaromyces*

*emersonii.*

*Activity*

A polypeptide of the invention can have one or more of the following features, namely it:

> (1) possesses β-glucanase (or cellulase) activity;
> (2) has an optimum pH range of from 3 to 6.5 or 7.0, such as from 4 to 5.5 or 6.0, optimally from 4.5 to 5.0;
> (3) has optimum activity at a temperature of from 30°C to 100°C, such as 60 to 95°C, optimally from 75°C or 80°C to 90°C. Preferably the optimum temperature is at least 75°C, such as at least 85°C;
> (4) has a molecular weight (deglycosylated) of from 20 to 60 kDa, preferably from 20 to 25, 3 5 to 45 or 23 to 50 kDa, optimally from 36 to 40 kDa or (glycosylated) or from 40 to 45kDa;
> (5) has an isoelectric point of from 3.0 to 3.6 or 5.0, 3.8 to 4.5, 4.5 to 5.0 or from 6.0 to 7.0; and/or
> (6) possesses hydrolytic activity on cereal β-glucan or exhibits hydrolytic activity below pH 7.

The polypeptide can have the activity of EC.3.2.1.4 (or endoglucanase activity). In general this can be endohydrolysis of 1,4-β-D-glucosidic linkages in cellulose. "β-glucanase activity" is the ability to cleave cellulose or a β-glucan polymer (for example as found in plants e.g. oat or barley). The activity thus allows cleavage between adjacent glucopyranose terminal and/or non-terminal units. Preferably the cleavage occurs at a [glucose (1-4), (1-3) or (1-6) glucose] linkage. The polypeptide may preferentially cleave in between two adjacent (e.g. non-substituted glucose) units. It can thus have endo activity (i.e. be an endoglucanase). The substrate polymer may or may not be substituted. Preferably the polypeptide will not have xylanase activity.

The polypeptide may also have the cellulase activity, that is to say it is active on, or can cleave, cellulose. As β-glucan is a component of cellulose, all glucanases fall within the broader term of cellulases. The polypeptides of the present invention are therefore cellulases because they belong in the sub-class of glucanases. Other types of classes of activity within cellulases, apart from endoglucanase (EC 3.2.1.4, as mentioned above) are exo-glucanase/cellobiohydrolase (EC 3.2.1.91), β-glucosidase (EC 3.2.1.21), endo-1,6-glucanase (EC 3.2.1.75), exo-1,3-glucanase (EC 3.2.1.58), mannanase (EC 3.2.1.78) and endo-glycoceramidase (EC 3.2.1.123).. Some of the polypeptides are thought to have one, two or more of these additional activities based on their structure and sequence, by comparison with known enzymes and the family of hydrolases into which they fall. Thus, in the specification, where the context is appropriate, glucanase activity can mean cellulase activity. A list of (cellulase) activities for the polypeptides is provided in Example 11 later.

Preferably the polypeptide falls into one of the families 5, 7 or 45, according to the glycoside hydrolase (CAZy) classification. The polypeptide may be a glu/glu or asp/asp nucleophile/proton donor.

*Variants and Homologues*

A polypeptide of the invention can comprise the amino acid sequence set out in SEQ ID No. 4 or a substantially homologous sequence, or a fragment of either sequence and can have β-glucanase activity. In general, the naturally occurring amino acid sequence shown in SEQ ID No. 4 is preferred.

In particular, the polypeptide of the invention may comprise:

> a. the polypeptide sequence of SEQ ID No. 4;
> b. a naturally occurring variant or species homologue thereof; or
> c. a protein with at least 70, at least 80, at least 90, at least 95, at least 98 or at least 99% sequence identity to (a) or (b).

A variant may be one that occurs naturally, for example in fungal, bacteria, yeast or plant cells and which can function in a substantially similar manner to the protein of SEQ ID No. 4, for example it has β-glucanase activity. Similarly a species homologue of the protein will be the equivalent protein which occurs naturally in another species and which can function as an β-glucanase enzyme. Variants include allelic variants either from the same strain as the polypeptide of the invention or from a different strain, but of the same genus, or of the same species.

Variants and species homologues can be obtained by following the procedures described herein for the production of the polypeptide of SEQ ID No. 4 and performing such procedures on a suitable cell source, for example a bacterial, yeast, fungal or plant cell. It will also be possible to use a probe as defined above to probe libraries made from yeast, bacterial, fungal or plant cells in order to obtain clones including the variants or species homology. The clones can be manipulated by conventional techniques to generate a polypeptide of the invention which can then be produced by recombinant or synthetic techniques known *per se*.

The polypeptide of the invention preferably has at least 70% sequence identity to the protein of SEQ ID No. 4, more preferably at least 80%, at least 90%, at least 95%, at east 97% or at least 99% sequence identity thereto, for example over a region of at least 60, at least 100, 150, 200 or 300 contiguous amino acids or over the full length of SEQ ID No. 4. For SEQ ID No. 4, the sequence identity is preferably at least 60%.

The sequence of the polypeptide of SEQ ID No. 4 and of variants and species homologues can thus be modified to provide polypeptides of the invention. Amino acid substitutions may be made, for example from or up to 1, 2 or 3 to 10, 20, 30, 50 or 100 substitutions. The same number of deletions and insertions may also be made. These changes may be made outside regions critical to the function of the polypeptide and so may still result in an active enzyme. The modified polypeptide generally retains activity as a β-glucanase.

Polypeptides of the invention include fragments of the above mentioned full length polypeptides and of variants thereof, including fragments of the sequence set out in SEQ ID No. 4. Such fragments typically retain activity as a β-glucanase. Fragments may be at least 50, 100, 150, 200 or 250 amino acids long or may be this number of amino acids short of the full length sequence (as shown in SEQ ID No. 4).

Polypeptides of the invention can if necessary be produced by synthetic means although usually they will be made recombinantly as described below. They may be modified for example by the addition of histidine residues or a T7 tag to assist their identification or purification or by the addition of a signal sequence to promote their secretion from a cell.

The term "variants" refers to polypeptides which have the same essential character or basic biological functionality as the β-glucanase (or cellulase), and include allelic variants. The essential character of β-glucanase is that it is an enzyme that exhibits EC 3.2.1.4 activity or that it can cleave 1→3, 1→4 and/or 1→6 links in β-D-glucan, such as cereal or oat spelt (β)-glucan. Preferably a variant polypeptide has the same activity as the β-glucanase. A polypeptide having the same essential character as β-glucanase may be identified by using a cellulose or β-glucan degradation assay, for example as described later.

Variants of SEQ ID No. 4 also include sequences which vary from SEQ ID No. 4 but which are not necessarily derived from the naturally occurring β-glucanase protein. These variants may be described as having a % homology to SEQ ID No. 4 or having a number of substitutions within this sequence. Alternatively a variant may be encoded by a polynucleotide which hybridizes to SEQ ID No. 3.

The variants can be defined in a similar manner to the variants of SEQ ID No. 3. Thus the variants may comprise variant sequences derived from other strains of *Talaromyces*. Other variants can be identified from other *Talaromyces* strains by looking for β-D-glucanase activity and cloning and sequencing as before. Variants may include the deletion, modification or addition of single amino acids or groups of amino acids within the protein sequence, as long as the peptide maintains the basic biological functionality of the β-glucanase.

Conservative substitutions may be made, for example according to the following Table. Amino acids in the same block in the second column and preferably in the same line in the third column may be substituted for each other. Preferably substitutions do not affect the folding or activity of the polypeptide.

| ALIPHATIC | Non-polar | G A P |
| | | I L V |
| | Polar-uncharged | C S T M |
| | | N Q |
| | Polar-charged | D E |
| | | K R |
| AROMATIC | | H F W Y |

Shorter polypeptide sequences are within the scope of the invention. For example, a peptide of at least 50 amino acids or up to 60, 70, 80, 100, 150 or 200 amino acids in length is considered to fall within the scope of the invention as long as it demonstrates the basic biological functionality of the β-glucanase. In particular, but not exclusively, this aspect of the invention encompasses the situation when the protein is a fragment of the complete protein sequence and may comprise or represent a β-D-glucan (or mannose) binding region or a β-D-glucan (or cellulose) cleaving region.

*Modifications*

Polypeptides of the invention may be chemically modified, e.g. post-translationally modified. For example, they may be glycosylated (one or more times, by the same or different sugars) or comprise modified amino acid residues. They may also be modified by the addition of histidine residues (to assist their purification) or by the addition of a signal sequence (to promote insertion into the cell membrane). The polypeptide may have one or more (N) amino- or (C) carboxyl-terminal extensions, such as an amino-terminal methionine residue, a small linker peptide of up to about 20-25 residues, or a (small) extension that facilitates purification, such as a poly-histidine or T7 tag, an antigenic epitope or a (e.g. maltose) binding domain[14] (e.g. at the C-terminus). These extensions may or may not be added via a linker.

A polypeptide of the invention may be labelled with a revealing labeL The revealing label may be any suitable label which allows the polypeptide to be detected. Suitable labels include radioisotopes, e.g. $^{125}$I,$^{35}$S, enzymes, antibodies, polynucleotides and linkers such as biotin.

The polypeptides may be modified to include non-naturally occurring amino acids or to increase the stability of the polypeptide. When the proteins or peptides are produced by synthetic means, such amino acids may be introduced during production. The proteins or peptides may also be modified following either synthetic or recombinant production.

The polypeptides of the invention may also be produced using, or comprise, (one or more) D-amino acids.

A number of side chain modifications are known in the art and may be made to the side chains of the proteins or peptides of the present invention. Such modifications include, for example, modifications of amino acids by reductive alkylation by reaction with an aldehyde followed by reduction with $NaBH_4$, amidination with methylacetimidate or acylation with acetic anhydride.

The sequences provided by the present invention may also be used as starting materials for the construction of "second generation" enzymes. "Second generation" glucanases are glucanases, altered by mutagenesis techniques (e.g. site-directed mutagenesis), which have properties that differ from those of wild-type glucanases or recombinant glucanases such as those produced by the present invention. For example, the temperature or pH optimum, specific activity, substrate affinity or thermostability may be altered so as to be better suited for application in a defined process.

Amino acids essential to the activity of the glucanases of the invention, and therefore preferably subject to substitution, may be identified according to procedures known in the art, such as site-directed mutagenesis or alanine-scanning mutagenesis[10]. In the latter technique mutations are introduced at every residue in the molecule, and the resultant mutant molecules are tested for biological activity (e.g. glucanase activity) to identify amino acid residues that are critical to the activity of the molecule. Sites of enzyme-substrate interaction can also be determined by analysis of crystal structure as determined by such techniques as nuclear magnetic resonance, crystallography or photo-affinity labelling[11,12,13] or molecular modelling:

The use of yeast and fungal host cells is expected to provide for such post-tmnslational modifications (e.g. proteolytic processing, myristilation, glycosylation, truncation, and tyrosine, serine or threonine phosphorylation) as may be needed to confer optimal biological activity on recombinant expression products of the invention.

Polypeptides of the invention may be provided in a form such that they are outside their natural cellular environment Thus, they may be substantially isolated or purified, as discussed above, or in a cell in which they do not occur in nature, e.g. a cell of other fungal species, animals, yeast or bacteria.

C. Recombinant Aspects.

The invention also provides vectors comprising a polynucleotide of the invention, including cloning and expression vectors, and methods of growing, transforming or transfecting such vectors in a suitable host cell, for example under conditions in which expression of a polypeptide of the invention occurs, Provided also are host cells comprising a polynucleotide or vector of the invention wherein the polynucleotide is heterologous to the genome of the host cell. The term "heterologous", usually with respect to the host cell, means that the polynucleotide does not naturally occur in the genome of the host cell or that the polypeptide is not naturally produced by that cell. Preferably, the host cell is a yeast cell, for example a yeast cell of the genus *Kluyveromyces* or *Saccharomyces* or a fungal cell, for example of the genus *Aspergillus*.

Polynucleotides of the invention can be incorporated into a recombinant replicable vector, for example a cloning or expression vector. The vector may be used to replicate the nucleic acid in a compatible host cell. Thus in a further embodiment, the invention provides a method of making polynucleotides of the invention by introducing polynucleotide of the invention into a replicable vector, introducing the vector into a compatible host cell, and growing the host cell under conditions which bring about replication of the vector. The vector may be recovered from the host cell. Suitable host cells are described below in connection with expression vectors.

*Vectors*

The polynucleotide of the invention may inserted into an expression cassette. The vector into which the expression cassette or polynucleotide of the invention is inserted may be any vector which may conveniently be subjected to recombinant DNA procedures, and the choice of the vector will often depend on the host cell into which it is to be introduced. Thus, the vector may be an autonomously replicating vector, i.e. a vector which exists as an extra-chromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

Preferably, a polynucleotide of the invention in a vector is operably linked to a regulatory sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence such as a promoter, enhancer or other expression regulation signal "operably linked" to a coding sequence is positioned in such a way that expression of the coding sequence is achieved under condition compatible with the control sequences.

The vector may be a plasmid, cosmid, virus or phage vector, usually provided with an origin of replication, optionally a promoter for the expression of the polynucleotide and optionally an enhancer and/or a regulator of the promoter. A terminator sequence may be present, as may be a polyadenylation sequence. The vector may contain one or more selectable marker genes, for example an ampicillin resistance gene (in the case of a bacterial plasmid) or a neomycin resistance gene (for a mammalian vector). Vectors may be used *in vitro,* for example for the production of RNA or used to transfect or transform a host cell. They may comprise two or more polynucleotides of the invention, for example for overexpression.

The DNA sequence encoding the polypeptide is preferably introduced into a suitable host as part of an expression cassette (or construct) in which the DNA sequence is operably linked to expression signals which are capable of directing expression of the DNA sequence in the host cells. For transformation of the suitable host with the expression construct transformation procedures are available which are well known to the skilled person[3,4]. The expression construct can be used for transformation of the host as part of a vector carrying a selectable marker, or the expression construct may be co-transformed as a separate molecule together with the vector carrying a selectable marker. The vector may comprise one or more selectable marker genes.

Preferred selectable markers[15,16] include but are not limited to those that complement a defect in the host cell or confer resistance to a drug. They include e.g. versatile marker genes that can be used for transformation of most filamentous fungi and yeasts such as acetamidase genes or cDNAs (the *amdS, niaD, facA* genes or cDNAs from *A. nidulans, A.oryzae,* or *A.niger*), or genes providing resistance to antibiotics like G418, hygromycin, bleomycin, kanamycin, phleomycin or benomyl resistance (benA). Alternatively, specific selection markers can be used such as auxotrophic markers which require corresponding mutant host strains: e.g. *URA3* (from *S.cerevisiae* or analogous genes from other yeasts), *pyrG* or *pyrA* (from *A.nidulans* or *A. niger*), *argB* (from *A. nidulans* or *A. niger*) or *trpC*. In a preferred embodiment the selection marker is deleted from the transformed host cell after introduction of the expression construct so as to obtain transformed host cells capable of producing the polypeptide which are free of selection marker genes[21,22].

Other markers include ATP synthetase, subunit 9 (*oli*C), orotidine-5' phospbate= decarboxylase (*pvr*A), the bacterial G418 resistance gene (this may also be used in yeast, but not in fungi), the ampicillin resistance gene (*E. coli*), the neomycin resistance gene (*Bacillus*) and the *E. coli uid*A gene, coding for β-glucuronidase (GUS). Vectors may be used *in vitro,* for example for the production of RNA or used to transfect or transform a host cell.

For most filamentous fungi and yeast, the vector or expression construct is preferably integrated in the genome of the host cell in order to obtain stable transformants. However, for certain yeasts also suitable episomal vectors are available into which the expression construct can be incorporated for stable and high level expression, examples thereof include vectors derived from the 2μ and pKD1 plasmids of *Saccharomyces* and *Kluyveromyces*, respectively, or vectors containing an AMA sequence (e.g. AMA1 from *Aspergillus*[3,20]). In case the expression constructs are integrated in the host cells genome, the constructs are either integrated at random loci in the genome, or at predetermined target loci using homologous recombination, in which case the target loci preferably comprise a highly expressed gene. A highly expressed gene is a gene whose mRNA can make up at least 0.01% (w/w) of the total cellular mRNA, e.g. under induced conditions, or alternatively, a gene whose gene product can make up at least 0.2% (w/w) of the total cellular protein, or, in case of a secreted gene product, can be secreted to a level of at least 0.05g/l. A number of examples of suitable highly expressed genes are provided below.

A vector or expression construct for a given host cell may comprise the following elements, operably linked to each other in a consecutive order from the 5'-end to 3'-end relative to the coding strand of the sequence encoding the polypeptide of the first invention:

> (1) a promoter sequence capable of directing transcription of the DNA sequence encoding the polypeptide in the given host cell;
> (2) optionally, a signal sequence capable of directing secretion of the polypeptide from the given host cell into a culture medium;
> (3) a DNA sequence encoding a mature and preferably active form of the polypeptide; and preferably also
> (4) a transcription termination region (terminator) capable of terminating transcription downstream of the DNA sequence encoding the polypeptide.

Downstream of the DNA sequence encoding the polypeptide there may be a 3' untranslated region containing one or more transcription termination sites (e.g. a terminator). The origin of the terminator is less critical. The terminator can e.g. be native to the DNA sequence encoding the polypeptide. However, preferably a yeast terminator is used in yeast host cells and a filamentous fungal terminator is used in filamentous fungal host cells. More preferably, the terminator is endogenous to the host cell (in which the DNA sequence encoding the polypeptide is to be expressed).

Enhanced expression of the polynucleotide encoding the polypeptide of the invention may also be achieved by the selection of heterologous regulatory regions, e.g. promoter, secretion leader and/or terminator regions, which may serve to increase expression and, if desired, secretion levels of the protein of interest from the expression host and/or to provide for the inducible control of the expression of the polypeptide of the invention.

Aside from the promoter native to the gene encoding the polypeptide of the invention, other promoters may be used to direct expression of the polypeptide of the invention. The promoter may be selected for its efficiency in directing the expression of the polypeptide of the invention in the desired expression host.

Promoters/enhancers and other expression regulation signals may be selected to be compatible with the host cell for which the expression vector is designed. For example prokaryotic promoters may be used, in particular those suitable for use in *E. coli* strains. When expression is carried out in mammalian cells, mammalian promoters may be used.. Tissues-specific promoters, for example hepatocyte cell-specific promoters, may also be used. Viral promoters may also be used, for example the Moloney murine leukaemia virus long terminal repeat (MMLV LTR), promoter rous sarcoma virus (RSV) LTR promoter, SV40 (e.g. large T antigen) promoter, human cytomegalovirus (CMV) IE promoter, herpes simplex virus promoters or adenovirus promoters, HSV promoters such as the HSV IE promoters), or HPV promoters, particularly the HPV upstream regulatory region (URR).. Yeast promoters include *S. cerevisiae* GAL4 and ADH promoters, the *S. pombe nmt* 1 and *adh* promoter. Mammalian promoters include the metallothionein promoter which may be induced in response to heavy metals such as cadmium and β-actin promoters. Tissue-specific promoters, in particular endothelial or neuronal cell specific promoters (for example the DDAHI and DDAHII promoters), are especially preferred.

A variety of promoters[15,16] can be used that are capable of directing transcription in the host cells of the invention. Preferably the promoter sequence is derived from a highly expressed gene as previously defined. Examples of preferred highly expressed genes from which promoters are preferably derived and/or which are comprised in preferred predetermined target loci for integration of expression constructs, include but are not limited to genes encoding glycolytic enzymes such as triose-phosphate isomerases (TPI), glyceraldehyde-phosphate dehydrogenases (GAPDH), phosphoglycerate kinases (PGK), pyruvate kinases (PYK), alcohol dehydrogenases. (ADH), as well as genes encoding amylases, glucoamylases, proteases, xylanases, cellobiohydrolases, β-galactosidases, alcohol (methanol) oxidases, elongation factors and ribosomal proteins. Specific examples of suitable highly expressed genes include e.g. the *LAC4* gene from *Kluyveromyces* sp., the methanol oxidase genes (*AOX* and *MOX*) from *Hansenula* and *Pichia*, respectively, the glucoamylase (*glaA*) genes *from A.niger* and *A.awamori*, the *A.oryzae* TAKA-amylase gene, the *A.nidulans gpdA* gene and the *T.reesei* cellobiohydrolase genes.

Examples of strong constitutive and/or inducible promoters which are preferred for use in fungal expression hosts[15,16] are those which are obtainable from the fungal genes for xylanase (*xln*A), phytase, ATP-synthetase, subunit 9 (*oli*C), triose phosphate isomerase(*tpi*), alcohol dehydrogenase (*Adh*A), α-amylase (*amy*), amyloglucosidase (AG - from the *gla*A gene), acetamidase (*amd*S) and glyceraldehyde-3-phosphate dehydrogenase (*gpd*) promoters.

Examples of strong yeast promoters are those obtainable from the genes for alcohol dehydrogenase, lactase, 3-phosphoglycerate kinase and triosephosphate isomerase.

Examples of strong bacterial promoters are the α-amylase and *SPo2* promoters as well as promoters from extracellular protease genes.

Promoters suitable for plant cells include napaline synthase (nos), octopine synthase (ocs), mannopine synthase (mas), ribulose small subunit (rubisco ssu), histone, rice actin, phaseolin, cauliflower mosaic virus (CMV) 35S and 19S and circovirus promoters. All these promoters are readily available in the art.

The vector may further include sequences flanking the polynucleotide giving rise to RNA which comprise sequences homologous to eukaryotic genomic sequences, preferably mammalian genomic sequences, or viral genomic sequences. This will allow the introduction of the polynucleotides of the invention into the genome of eukaryotic cells or viruses by homologous recombination. In particular, a plasmid vector comprising the expression cassette flanked by viral sequences can be used to prepare a viral vector suitable for delivering the polynucleotides of the invention to a mammalian cell. Other examples of suitable viral vectors include herpes simplex viral vectors[18,19] and retroviruses, including lentiviruses, adenoviruses, adeno-associated viruses and HPV viruses (such as HPV-16 or HPV-18). Gene transfer techniques using these viruses are known to those skilled in the art. Retrovirus vectors for example may be used to stably integrate the polynucleotide giving rise to the antisense RNA into the host genome. Replication-defective adenovirus vectors by contrast remain episomal and therefore allow transient expression.

The vector may contain a polynucleotide of the invention oriented in an antisense direction to provide for the production of antisense RNA. This may be used to reduce, if desirable, the levels of expression of the polypeptide.

*Host cells and Expression*

In a further aspect the invention provides a process for preparing a polypeptide according to the invention which comprises cultivating a host cell (e.g. transformed or transfected with an expression vector as described above) under conditions to provide for expression (by the vector) of a coding sequence encoding the polypeptide, and optionally recovering the expressed polypeptide. Polynucleotides of the invention can be incorporated into a recombinant replicable vector, e.g. an expression vector. The vector may be used to replicate the nucleic acid in a compatible host cell. Thus in a further embodiment, the invention provides a method of making a polynucleotide of the invention by introducing a polynucleotide of the invention into a replicable vector, introducing the vector into a compatible host cell, and growing the host cell under conditions which bring about the replication of the vector. The vector may be recovered from the host cell. Suitable host cells include bacteria such as *E. coli*, yeast, mammalian cell lines and other eukaryotic cell lines, for example insect cells such as Sf9 cells and (e.g. filamentous) fungal cells.

Preferably the polypeptide is produced as a secreted protein in which case the DNA sequence encoding a mature form of the polypeptide in the expression construct is operably linked to a DNA sequence encoding a signal sequence. Preferably the signal sequence is native (homologous) to the DNA sequence encoding the polypeptide. Alternatively the signal sequence is foreign (heterologous) to the DNA sequence encoding the polypeptide, in which case the signal sequence is preferably endogenous to the host cell in which the DNA sequence is expressed. Examples of suitable signal sequences for yeast host cells are the signal sequences derived from yeast α-factor genes. Similarly, a suitable signal sequence for filamentous fungal host cells is e.g. a signal sequence derived from a filamentous fungal amyloglucosidase (AG) gene, e.g. the *A.niger glaA* gene. This may be used in combination with the amyloglucosidase (also called (gluco)amylase) promoter itself, as well as in combination with other promoters. Hybrid signal sequences may also be used with the context of the present invention.

Preferred heterologous secretion leader sequences are those originating from the fungal amyloglucosidase (AG) gene (*gla*A - both 18 and 24 amino acid versions e.g. from *Aspergillus*), the α-factor gene (yeasts e.g. *Saccharomyces* and *Kluyveromyces*) or the α-amylase gene (*Bacillus*)

The vectors may be transformed or transfected into a suitable host cell as described above to provide for expression of a polypeptide of the invention. This process may comprise culturing a host cell transformed with an expression vector as described above under conditions to provide for expression by the vector of a coding sequence encoding the polypeptide.

A further aspect of the invention thus provides host cells transformed or transfected with or comprising a polynucleotide or vector of the invention. Preferably the polynucleotide is carried in a vector for the replication and expression of the polynucleotide. The cells will be chosen to be compatible with the said vector and may for example be prokaryotic (for example bacterial), fungal, yeast or plant cells.

A heterologous host may also be chosen wherein the polypeptide of the invention is produced in a form which is substantially free from other cellulose-degrading enzymes. This may be achieved by choosing a host which does not normally produce such enzymes such as *Kluyveromyces lactis*.

The invention encompasses processes for the production of the polypeptide of the invention by means of recombinant expression of a DNA sequence encoding the polypeptide. For this purpose the DNA sequence of the invention can be used for gene amplification and/or exchange of expression signals, such as promoters, secretion signal sequences, in order to allow economic production of the polypeptide in a suitable homologous or heterologous host cell. A homologous host cell is herein defined as a host cell which is of the same species or which is a variant within the same species as the species from which the DNA sequence is derived.

Suitable host cells are preferably prokaryotic microorganisms such as bacteria, or more preferably eukaryotic organisms, for example fungi, such as yeasts or filamentous fungi, or plant cells. In general, yeast cells are preferred over fungal cells because they are easier to manipulate. However, some proteins are either poorly secreted from yeasts, or in some cases are not processed properly (e.g. hyperglycosylation in yeast). In these instances, a fungal host organism should be selected.

The host cell may over-express the polypeptide, and techniques for engineering over-expression are well known[3]. The host may thus have two or more copies of the encoding polynucleotide (and the vector may thus have two or more copies accordingly).

Bacteria from the genus *Bacillus* are very suitable as heterologous hosts because of their capability to secrete proteins into the culture medium. Other bacteria suitable as hosts are those from the genera *Streptomyces* and *Pseudomonas.* A preferred yeast host cell for the expression of the DNA sequence encoding the polypeptide is of the genera *Saccharomyces, Kluyveromyces, Hansenula, Pichia, Yarrowia,* and *Schizosaccharomyces.* More preferably a yeast host cell is selected from the group consisting of the species *Saccharomyces cerevisiae, Kluyveromyces lactis* (also known as *Kluyveromyces marxianus* var. *lactis*), *Hansenula polymorpha, Pichia pastoris, Yarrowia lipolytica,*and *Schizosaccharomyces pombe.*

Most preferred are, however, (e.g. filamentous) fungal host cells. Preferred filamentous fungal host cells are selected from the group consisting of the *genera Aspergillus, Trichoderma, Fusarium, Disporotrichum, Penicillium, Acremonium, Neurospora, Thermoascus, Myceliophtora, Sporotrichurn, Thielavia,* and *Talaromyces.* More preferably a filamentous fungal host cell is of the species *Aspergillus oyzae, Aspergillus sojae, Aspergillus nidulans,* or a species from the *Aspergillus niger* Group (as defined by Raper and Fennell, The Genus Aspergillus, The Williams & Wilkins Company, Baltimore, pp 293-344, 1965). These include but are not limited to *Aspergillus niger, Aspergillus awamori, Aspergillus tubigensis, Aspergillus aculeatus, Aspergillus foetidus, Aspergillus nidulans, Aspergillus japonicus, Aspergillus oryzae* and *Aspergillus ficuum,* and further consisting of the species *Trichoderma reesei, Fusarium graminearum, Penicillium chrysogenum, Acremonium alabamense, Neurospora crassa, Myceliophtora thermophilum, Sporotrichum cellulophilum, Disporotrichum dimorphosporum* and *Thielavia terrestris.*

Examples of preferred expression hosts within the scope of the present invention are fungi such as *Aspergillus* species (described in EP-A-184,438 and EP-A-284,603) and *Trichoderma* species; bacteria such as *Bacillus* species (described in EP-A-134,048 and EP-A-253,455), e.g. *Bacillus subtilis, Bacillus licheniformis, Bacillus amyloliquefaciens, Pseudomonas* species; and yeasts such as *Kluyveromyces* species (described in EP-A-096,430 e.g. *Kluyveromyces lactis* in EP-A-301,670) and *Saccharomyces* species, e.g. *Saccharomyces cerevisiae.*

Host cells according to the invention include plant cells, and the invention therefore extends to transgenic organisms, such as plants and parts thereof, which contain one or more cells of the invention. The cells may heterologously express the polypeptide of the invention or may heterologously contain one or more of the polynucleotides of the invention. The transgenic (or genetically modified) plant may therefore have inserted (e.g. stably) into its genome a sequence encoding one or more of the polypeptides of the invention. The transformation of plant cells can be performed using known techniques, for example using a Ti or a Ri plasmid from *Agrobacterium tumefaciens.* The plasmid (or vector) may thus contain sequences necessary to infect a plant, and derivatives of the Ti and/or Ri plasmids may be employed.

Alternatively direct infection of a part of a plant, such as a leaf, root or stem can be effected. In this technique the plant to be infected can be wounded, for example by cutting the plant with a razor or puncturing the plant with a needle or rubbing the plant with an abrasive. The wound is then innoculated with the *Agrobacterium.* The plant or plant part can then be grown on a suitable culture medium and allowed to develop into a mature plant Regeneration of transformed cells into genetically modified plants can be achieved by using known techniques, for example by selecting transformed shoots using an antibiotic and by sub-culturing the shoots on a medium containing the appropriate-nutrients, plant hormones and the like.[17]

*Culture of host cells and recombinant production*

The invention also includes cells that have been modified to express the β-glucanase or a variant thereof. Such cells include transient, or preferably stable higher eukaryotic cell lines, such as mammalian cells or insect cells, lower eukaryotic cells, such as yeast and (e.g. filamentous) fungal cells or prokaryotic cells such as bacterial cells.

It is also possible for the proteins of the invention to be transiently expressed in a cell line or on a membrane, such as for example in a baculovirus expression system. Such systems, which are adapted to express the proteins according to the invention, are also included within the scope of the present invention.

According to the present invention, the production of the polypeptide of the invention can be effected by the culturing of microbial expression hosts, which have been transformed with one or more polynucleotides of the present invention, in a conventional nutrient fermentation medium.

The recombinant host cells according to the invention may be cultured using procedures known in the art. For each combination of a promoter and a host cell, culture condition are available which are conducive to the expression the DNA sequence encoding the polypeptide. After reaching the desired cell density or titre of the polypeptide the culture is stopped and the polypeptide is recovered using known procedures.

The fermentation medium can comprise a known culture medium containing a carbon source (e.g. glucose, maltose, molasses, cellulose, β-glucan etc.), a nitrogen source (e.g. ammonium sulphate, ammonium nitrate, ammonium chloride, etc.); an organic nitrogen source (e.g. yeast extract, malt extract, peptone, etc.) and inorganic nutrient sources (e.g. phosphate, magnesium, potassium, zinc, iron, etc.). Optionally, an inducer (e.g. cellulose, β-glucan, maltose or maltodextrin) may be included.

The selection of the appropriate medium may be based on the choice of expression host and/or based on the regulatory requirements of the expression construct. Such media are known to those skilled in the art. The medium may, if desired, contain additional components favouring the transformed expression hosts over other potentially contaminating microorganisms.

The fermentation can be performed over a period of 0.5-30 days. It may be a batch, continuous or fed-batch process, suitably at a temperature in the range of between 0 and 45°C and, for example, at a pH between 2 and 10. Preferred fermentation conditions are a temperature in the range of between 20 and 37°C and/or a pH between 3 and 9. The appropriate conditions are usually selected based on the choice of the expression host and the protein to be expressed.

After fermentation, if necessary, the cells can be removed from the fermentation broth by means of centrifugation or filtration. After fermentation has stopped or after removal of the cells, the polypeptide of the invention may then be recovered and, if desired, purified and isolated by conventional means.

D. Uses of the polypeptides and methods of processing plant or cellulose-containing materials

The polypeptides of the invention which possess β-glucanase (or cellulase) activity may be used to treat fungal or plant material including plant pulp and plant extracts. For example, they may be used to treat cereals, vegetables, fruits or extracts thereof. They may be used in washing compositions (liquid or solid, e.g. powder) and/or in detergent compositions. Conveniently the polypeptide of the invention is combined with suitable (solid or liquid) carriers or diluents including buffers to produce a composition/ enzyme preparation. The polypeptide may be attached to or mixed with a carrier, e.g. immobilized on a solid carrier. Thus the present invention provides in a further aspect a composition comprising a polypeptide of the invention. This may be in a form suitable for packaging, transport and/or storage, preferably where the glucanase activity is retained. Compositions include paste, liquid, emulsion, powder, flake, granulate, pellet or other extrudate form.

The composition may further comprise additional ingredients such as one or more enzymes, for example pectinases, including an .(e.g. endo)-arabinanase and rhamnogalacturonase, other cellulases, xylanases, galactanases, mannanases and/or xyloglucanases. The polypeptide is typically stably formulated either in liquid or dry form. Typically, the product is made as a composition which will optionally include, for example, a stabilising buffer and/or preservative. The compositions may also include other enzymes capable of digesting plant material or cellulose, for example other cellulases, e.g. (β-D-)glucanases. For certain applications, immobilization of the enzyme on a solid matrix or incorporation on or into solid carrier particles may be preferred. The composition may also include a variety of other plant material-degrading enzymes, for example (other) cellulases and other pectinases.

The polypeptides and compositions of the invention may therefore be used in a method of processing plant material to degrade or modify the cellulose constituents (e.g. β-D-glucan) of the cell walls of the plant or fungal material. Thus in a further aspect, the present invention provides a method of degrading or modifying a plant cell or cellulose which method comprises contacting the plant, fungal cell or cellulose with a polypeptide or composition of the invention.

The invention also provides a method of processing a plant material which method comprises contacting the plant material with a polypeptide or composition of the invention to degrade or modify the cellulose in the plant material. Preferably the plant material is a plant pulp or plant extract.

In particular, the degradation preferably comprises cleaving of ß-glucan subunits of a cellulose component of the plant cell wall. The plant material is preferably a cereal, vegetable, fruit or vegetable or fruit pulp or extract. The present invention further provides a processed plant material obtainable by contacting a plant material with a polypeptide or composition of the invention.

The present invention also provides a method for reducing the viscosity of a plant extract which method comprises contacting the plant extract with a polypeptide or composition of the invention in an amount effective in degrading cellulose (or β-D-glucan) contained in the plant extract.

Plant and cellulose-containing materials include plant pulp, parts of plants and plant extracts. In the context of this invention an extract from a plant material is any substance which can be derived from plant material by extraction (mechanical and/or chemical), processing or by other separation techniques. The extract may be juice, nectar, base, or concentrates made thereof. The polypeptide may be used in (e.g. total) liquefaction and/or (advanced) maceration, for exampel in preparing (fruit) juices. The plant material may comprise or be derived from vegetables, e.g., carrots, celery; onions, legumes or leguminous plants (soy, soybean, peas) or fruit, e.g., pome or seed fruit (apples, pears, quince etc.), grapes, tomatoes, citrus (orange, lemon, lime, mandarin), melons, prunes, cherries, black currants, redcurrants, raspberries, strawberries, cranberries, pineapple and other tropical fruits, trees and parts thereof (e.g. pollen, from pine trees), or cereal (oats, barley, wheat, maize, rice).

The polypeptides of the invention can thus be used to treat plant material including plant pulp and plant extracts. They may also be used to treat liquid or solid foodstuffs or edible foodstuff ingredients.

Typically, the polypeptides of the invention are used as a composition/ enzyme preparation as described above. The composition will generally be added to plant pulp obtainable by, for example mechanical processing such as crushing or milling plant material. Incubation of the composition with the plant will typically be carried out for at time of from 10 minutes to 5 hours, such as 30 minutes to 2 hours, preferably for about 1 hour. The processing temperature is preferably 10-55°C, e.g. from 15 to 25°C, optimally about 20°C and one can use 10-300g, preferably 30-70g, optimally about 50g of enzyme per ton of material to be treated. All the enzyme(s) or their compositions used may be added sequentially or at the same time to the plant pulp. Depending on the composition of the enzyme preparation the plant material may first be macerated (e.g. to a purée) or liquefied. Using the polypeptides of the invention processing parameters such as the yield of the extraction, viscosity of the extract and/or quality of the extract can be improved.

Alternatively, or in addition to the above, a polypeptide of the invention may be added to the raw juice obtained from pressing or liquefying the plant pulp. Treatment of the raw juice will be carried out in a similar manner to the plant pulp in respect of dosage, temperature and holding time. Again, other enzymes such as those discussed previously may be included. Typical incubation conditions are as described in the previous paragraph. Once the raw juice has been incubated with the polypeptides of the invention, the juice is then centrifuged or (ultra) filtered to produce the final product

A composition containing a polypeptide of the invention may also be used during the preparation of fruit or vegetable purees.

The polypeptide of the invention may also be used in brewing, wine making, distilling or baking. It may therefore used in the preparation of alcoholic beverages such as wine and beer, for example to improve the filterability or clarity of wine. In baking the polypeptide may improve the dough structure, modify its stickiness or suppleness, improve the loaf volume and/or crumb structure.

Polypeptide of the invention can be used in brewing. In brewing, filtration problems can arise in mash-bills, which include over-modified malts, due to the present of β-glucans released at high temperatures. The reduction in the speed of wort filtration can be one of the main problems encountered. Additonal problems are colloidal stability and haze formation in finished beer. These may be caused by the same carbohydrate complexes, especially during high gravity brewing. The polypeptides of the invention may be able to improve the filterability of the wort, for example after mashing. In this way, less wort liquor may be retained in the spent grains, and the yield of extract can be improved. A more efficient filtration can result in an increased efficiency of the brewhouse. Thus, in general, the polypeptides of the invention can be used to improve the filterability or filtration rate of the (e.g. finished) beer.

The polypeptides of the invention may also prevent or at least mitigate haze formation during beer manufacture. β-glucans found in the endosperm walls of cereals can be carried over into the finished beer. This may cause haze and loss of clarity. The polypeptides of the invention may prevent or at least mitigate accumulation of particles due to the hydrolysis of β-glucan. Hence the polypeptides of the invention may be used to increase the colloidal stability of the (e.g. finished) beer.

The polypeptides find use in a number of industrial areas due to their glucanase activity. These can include not only alcohol production, but also in biomethanation, in bread making and in baking, in dental hygiene (for example dental or oral compositions), in the treatment or fabric, clothes or manufacture of leather, in the manufacture of paper, in pharmaceuticals, in tea, in the preparation or treatment of textiles, in detergent or washing compositions, and in the treatment of waste. One aspect of the invention is therefore a food or foodstuff comprising the polypeptide, such as an alcoholic beverage, bread, dough or tea. The polypeptide may be formulated into a suitable compositions for any of these uses. The polypeptide may be present in an aqueous composition (eg. hot water), preferably with one or more fungicides, in order to treat plant material (eg. bulbs), especially to control parasitic insects, mites and nematodes.

As the polypeptides of the invention can degrade glucan it may be added to foods or foodstuffs (for example by consumption by humans). It is known that soluble β-D-glucan is associated with the lowering of serum cholesterol and triglycerides, and therefore the polypeptides of the invention can be used to lower serum cholesterol and triglyceride levels in humans or animals, for example in hyperlipemic individuals. The invention therefore includes pharmaceutical and veterinary compositions that comprise the polypeptide of the invention and the pharmaceutically or veterinarily acceptable carrier. The polypeptides can thus be used in the manufacture of a medicament for preventing or treating hyperlipemia, or high serum cholesterol and triglyceride levels or disorders resulting therefrom.

Polypeptides of the invention may also display anti-fungal activity. They may be able to degrade fungal cell walls, and thus can be employed for fungal cell wall lysis, in order to open the cells. This may release intracellular proteins. In such a way the polypeptides may be used to prepare yeast and/or fungal extracts.

E. Animal Feeds

The invention additionally relates to foodstuffs or an animal feed composition comprising one or more polypeptides of the invention. The polypeptide may be present in the feed at a concentration different from its natural concentration. Preferred amounts are from 0.1 to 100, such as 0.5 to 50, preferably 1 to 10, mg per kg feed.

The invention also relates to a process for the preparation of an animal feed composition, the process comprising adding to one or more edible feed substance(s) or ingredient(s) a polypeptide of the invention. The polypeptides can be added to the animal feed composition separately from the feed substances or ingredients, individually or in combination with other feed additives. The polypeptide can be an integral part of one of the feed substances or ingredients.

The polypeptides of the invention may also be added to animal feeds rich in cellulose to improve the breakdown of the plant cell wall leading to improved utilisation of the plant nutrients by the animal. The polypeptides of the invention may be added to the feed or silage if pre-soaking or wet diets are preferred. Advantageously, the polypeptides of the invention may continue to degrade cellulose in the feed *in vivo*. Fungal derived polypeptides of the invention in particular generally have lower pH optima and are capable of releasing important nutrients in such acidic environments as the stomach of an animal. The invention thus also contemplates (e.g. animal) feeds or foodstuffs comprising one or more polypeptides of the invention.

The polypeptides of the invention may also be used during the production of milk substitutes (or replacers) from soy bean. These milk substitutes can be consumed by both humans and animals. A typical problem during the preparation of these milk substitutes is the high viscosity of the soy bean slurry, resulting in the need for an undesirable dilution of the slurry to a concentration of dry solids of 10 to 15%. An enzyme preparation containing a polypeptide of the invention can be added to, or during the processing of, the slurry, enabling processing at a higher concentration (typically 40 to 50%) dry solids. The enzyme may also be used in the preparation of savoury product(s), e.g. from soy bean.

The composition may additionally comprise (particularly when being formulated for use in animal feed) one or more ionophores, oxidising agents, surfactants, rumen protected amino acids, enzyme enhancers or enzymes which may be produced naturally in the gastro-intestinal tract of the animals to be fed.

When added to feeds (including silage) for ruminants or monogastric animals (eg. poultry or swine) the feeds may comprise cereals such as barley, wheat, maize, rye or oats or cereal by-products such as wheat bran or maize bran, or other plant materials such as soy beans and other legumes. The enzyme(s) may significantly improve the breakdown of plant cell walls which leads to better utilisation of the plant nutrients by the animal. As a consequence, growth rate and/or feed conversion may be improved. The polypeptides of the invention may be added to the feed (directly or as an additive or ingredient) or treated cellulose (e.g. glucan) may be added instead.

A particularly preferred method for the (exogenous) addition of the β-glucanase is to add the polypeptide of the invention as transgenic plant material and/or (e.g. transgenic) seed. The polypeptide may thus have been synthesized through heterologous gene expression, for example the gene encoding the desired enzyme may be cloned in to a plant expression vector, under control of the appropriate plant expression signals, e.g. a tissue specific promoter, such as a seed specific promoter. The expression vector containing the gene encoding the polypeptide can be subsequently transformed into plant cells, and transformed cells can be selected for regeneration into whole plants. The thus obtained transgenic plants can be grown and harvested, and those parts of the plants containing the heterologous (to the plant) polypeptide can be included in one of the compositions, either as such or after further processing. General methods for the (heterologous) expression of enzymes in (transgenic) plants, including methods for seed-specific expression of enzymes, are known[23]. The heterologous polypeptide may be contained in the seed of the transgenic plants or it may be contained in other plant parts such as roots, stems, leaves, wood, flowers, bark and/or fruit. The plant may be a monocot or a dicot Suitable plants include cereals, such as oats, barley, wheat, maize and rice. Preferably the polynucleotide of the invention is stably incorporated into the plant genome.

The addition of the polypeptide in the form of transgenic plant material, e.g. in transgenic seed may require the processing of the plant material so as to make the enzyme available, or at least improve its availability. Such processing techniques may include various mechanical (eg. milling and/or grinding) techniques or thermomechanical treatments such as extrusion or expansion.

The present invention thus also relates to a process for promoting growth and/or feed conversion in a monogastric or non-ruminant animal, the process comprising feeding the animal polypeptide of the invention. Suitable animals include farm, monogastric and/or non-ruminant animals such as pigs (or piglets), poultry (such as chickens, turkeys), calves or veal or aquatic (e.g. marine) animals (for example fish).

Assays for Cellulose Degrading Enzymes

Also within the present invention is the use of polypeptides according to the invention in screening methods to identify compounds that may act as agonists or antagonists which may modulate the β-glucanase. In general terms, such screening methods may involve contacting a polypeptide of the invention with a test compound and then measuring activity or incubating a polypeptide of the invention with a test substance and then detecting any modulation of β-glucanase activity. Agents which bind to the polypeptides of the present invention can also be identified by binding assays.

Modulator activity can be determined by contacting cells expressing a polypeptide of the invention with a substance under investigation and by monitoring the effect mediated by the polypeptides. The cells expressing the polypeptide may be *in vitro* and preferably, the assay is carried out *in vitro* using cells expressing recombinant polypeptide.

The assays and substrates described herein have allowed identification and confirmation of ß-glucanase activity. However, these assays can be used to detect other cellulose degrading enzymes, whether or not they have β-glucanase activity. The substrate that can be used for this assay can comprise ß-glucan.

Another aspect of the invention relates to an assay for identifying or detecting a polypeptide which is able to degrade cellulose. The activity may be a glucanase (e.g. ß-glucanase) or cellulase or xyloglucanase. The assay may comprise:

(a) providing, as a substrate for a candidate compound (usually a polypeptide) the substrate described in the previous paragraph; and

(b) contacting the substrate with the candidate compound, and detecting whether any carbohydrates are produced.

The amount of these carbohydrates can be measured. If necessary, they can then be compared to the amount of the carbohydrates produced in a control experiment, in the absence of candidate compound.

The above assays can be employed to identify modulators of the β-glucanase activity.

Preferred features and characteristics of one aspect of the invention are applicable to another aspect *mutatis mutandis*.

The invention will now be described with reference to the following Examples which are intended to be illustrative only and not limiting.

EXAMPLES

General procedures

Standard molecular cloning techniques such as DNA isolation, gel electrophoresis, enzymatic restriction modifications of nucleic acids, Southern analyses, *E. coli* transformation, colony lifts and filter hybridisations etc., were performed using standard techniques.[1,2] Synthetic oligo deoxynucleotides were obtained from ISOGEN Bioscience (Maarssen, The Netherlands). DNA sequence analyses were performed on an Applied Biosystems 373A DNA sequencer, according to the supplier's instructions.

DNA labelling and hybridizations were conducted according to the ECL™ direct nucleic acid labeling and detection systems (Amersham LIFE SCIENCE, Little Chalfont, England) or according to the standard radioactive labeling techniques.[1]

Example 1: RNA isolation from *T. emersonii* and synthesis of cDNA

*T. emersonii* strain CBS 393.64 was fermented under cellulase-inducing conditions. At several time points mycelium and culture supernatants were harvested by filtration using Miracloth filtration wrap. Mycelium was washed extensively with demineralized water and squeezed between paper towels to remove excessive water. Mycelium from selected time points (based on the cellulase measurements in culture supernatants) was frozen immediately in liquid nitrogen and ground to a fine powder using a mortar and pestle. The resulting powder was transferred to a sterile 50 ml tube and weighed: for every 1-1.2 g of ground mycelium 10 ml TRIzol reagent (Gibco/BRL) was added (max. 25 ml per tube). The mycelial powder was immediately solubilised by vigorous mixing (vortexing, 1 min), followed by 5 minutes at room temperature incubation with occasional mixing. A 0.2 (original TRIzol) volume of chloroform (thus 2 ml for every 10 ml TRIzol used originally) was added, vortexed and left at room temperature for 10 min. Subsequently, the mixture was centrifuged at 4°C, 6000 g for 30 minutes. The top aqueous phase was transferred to a fresh tube and total RNA was precipitated by addition of a 0.5 (original TRIzol) volume of isopropyl alcohol (thus 5 ml of isopropyl alcohol for every 10 ml TRIzol used originally). After 10 minutes of precipitation at room temperature, the RNA was recovered by centrifugation for 30 minutes at 6000 g. On removal of supernatant the RNA pellet was rinsed with one volume of 70% ethanol. After removal of the ethanol, the RNA pellet was air dried. The dried RNA pellet was dissolved in 3 ml GTS (100 mM Tris-Cl, pH 7.5,4 M guanidium thiocyanate, 0.5 % sodium lauryl sarcosinate) buffer. 10 μl of RNA solution was used to determine quality and concentration of nucleic acids.

Northern analysis was performed[3] and the isolated RNA further purified.[1,3] For isolation of mRNA a modified protocol (using gravity flow instead of centrifugation) of the PHARMACIA purification kit (Cat no. 27-9258-02) was used.[3] For cDNA synthesis the STRATAGENE cDNA Synthesis KIT was used according to the instructions of the manufacturer, except for a number of optimisations for using the pGBFIN vectors with major changes as previously described.[3]

The amount of cDNA synthesised was estimated by TCA precipitation and subsequently analysed via electrophoresis in alkaline agarose gels.[3]

Example 2: Preparation of a cDNA library from *T. emersonii* mRNA

The cDNA pool obtained in Example 1 was blunted, ligated with adapters and restriction enzyme digested.[3]

Cloning of the cDNA in the expression vector pGBFIN-11 (see WO-99/32617 for the construction of this vector) requires the presence of a EcoRI site on the 5'- and of an *Xho*I site on the 3'- end of the cDNA. Therefore, the first strand priming oligoriucleotide and the adapter sequences used (Pharmacia) were chosen to meet the prerequisites set for the expression vector.

The cDNAs obtained were separated via size fractionation through a SEPHAROSE CL-2B matrix, upon which size of the individual pools obtained were analysed via non-denaturing gel electrophoresis.[3] Two pools of cDNAs, obtained via out offs at 0.5 kb and 1.0 kb respectively, were selected for construction of the a cDNA library in pGBFIN-11. For the pGBFIN-11, a pool of completely double-digested (*Eco*RI-*Xho*I) pGBFIN-11 vector (background ligation < 1%) was prepared. The selected cDNA pools were ligated into the pGBFIN-11 vector and transformed into *E. coli* XL10-Gold bacterial cells to generate two primary cDNA libraries. Transformation frequencies of the two pools were both >1.0 x $10^6$. From a fraction of both the *E. coli* cDNA libraries, colonies were selected randomly and plasmid DNA was isolated. Analysis of this plasmid DNA demonstrated that both cDNA libraries had insert percentages between 90 and 95%.

Furthermore, colony lifts were performed from a fraction of the library and the generated filters were subsequently hybridised with the *T. emersonii gpdA* gene, encoding the glyceraldehyde-3-phosphate dehydrogenase gene. Next, plasmid DNA was isolated and via restriction analysis it was demonstrated that all plasmid contained single inserts in the correct orientation. Sequencing of the 5' ends of the cDNAs within these *T. emersonii gpdA* containing plasmids demonstrated that > 85% was full length.

Example 3: Transformation of the expression library to *A. niger*

DNA was isolated from the *E. coli* cDNA library as described earlier. Total plasmid DNA was digested for 4 hours at 37°C with *Not*I to remove *E. coli* derived plasmid sequences. After purification, the DNA was dissolved in sterile demineralised water.

Multiple *A. niger* DS2978 transformations were performed[3] using 1.5 x $10^7$ B 3.0 x $10^7$ protoplasts and 10 ug of plasmid DNA per transformation. Transformants were selected for the presence of the *amdS* selection marker by growth on acetamide as the sole N-source. Since both the *amdS* selection marker and the cDNA expression cassette are present on the integrating fragment growth on acetamide is indicative for the presence of a cDNA expression cassette.

After approximately 7-10 days incubation at 30°C, 10,000 transformants were purified: the *Aspergillus niger* transformants were transferred robotically (FlexysJcolony picker automater) from the transformation plates towards 96 wells MTP Master Plates (MPs) containing 150 $\mu$l per well of solidified selective medium (SM) (per 1000ml: 0.52 g KCl, 1.52 g $K_2$ HPO$_4$, 0.52 g MgSO$_4$, 20g glucose, 1g acetamide, 0.1M MES buffer, 15g agar, 1ml of trace element solution [trace elements solution (containing per 1 liter): 2.2g ZnSO4/7H$_2$O, 1.1g H$_3$BO$_3$, 0.5gFeSO$_4$/7H$_2$O, 0.17g CoCl$_2$/6H$_2$O, 0.16g CuSO$_4$/5H$_2$O, 0.15g NaMoO$_4$/2H$_2$O, 5.0g EDTA, pH 6.5] pH 5.5. The transformants were grown on SM for 5 days at 34°C. The thus generated set of MPs was used to 1) inoculate MTPs for growth and subsequent enzyme detection and 2) backup plates (BPs) of the cDNA library which were stored at -80°C.

Example 4: Analysis of the *T. emersonii* expression library

5 days-old grown MPs were used as replication template and replica plated on fresh selective medium (SM) plates, containing per litre: 0.52 g KCl, 1.52 g $K_2$HPO$_4$, 0.52 g MgSO$_4$, 20g glucose, 1g acetamide, 0.1M MES buffer, 15g agar 1ml of trace element solution [trace elements solution (per 1 litre): 22g ZnSO$_4$/7H$_2$O, 1.1g H$_3$BO$_3$, 0.5gFeSO$_4$/7H$_2$O, 0.17g CoCl$_2$/6H$_2$O, 0.16g CuSO$_4$/5H$_2$O, 0.15g NaMoO$_4$/2H$_2$O, 5.0g EDTA, pH 6.5] pH 5.5.

Once inoculated the plates were incubated at 34°C for 48h. Subsequently the plates were filled up with a top-agar-containing carboxymethylcellulose (CMC) (5g agarose, 0.5g CMC (Sigma ref C4888) prepared in 1000ml of 50 mM phosphate buffer pH 7). Once the top agar solidified, the plates were left at 65°C for 4 hours. For the activity visualization, the plates were stained with a Congo red solution (10 g Congo red in 1000ml phosphate buffer pH7) for 15 minutes. The staining solution was discarded and the plates were washed with 1M NaCl (58.44g in 1 litre of distilled water). This latter step was repeated twice. Positive clones appeared by forming a pale clearance halo on a red background.

The positive cellulase clones from this first screen (demonstrating a clear halo after congo red screening) were re-inoculated on fresh SM medium and grown for 5 days at 34°C. The thus-obtained template plate was then replicated on selective medium and on selective medium containing 0.075% (w/v) of AZCL-cellulose (Megazyme catalogue ref. I-AZCEL). The SM plates were treated and scored as described previously (growth at 34°C and subsequent screening via cellulose-containing overlay and Congo red straining) whereas the SM-AZCEL-cellulose plates were incubated at 34°C for 48 h and then further incubated at 65°C for 8 h. The SM-AZCL-cellulose plates were scored before and after the high temperature incubation. The positive cellulase clones resulted in a diffuse blue halo.

Finally, 20 positive cellulase clones were identified. Cellulase producing *Aspergillus* transformants, as identified in the xylanase plate assay, were grown in shake flask fermentation.[3] Medium samples were taken after 5 days of fermentation and analysed for cellulase activity as described later.

Example 5: Genetic analysis of positive transformants

Positive (re-confirmed) transformants identified were grown on liquid medium, the mycelium was harvested and total (chromosomal) DNA was isolated using the Puregene Isolation System (Biozym B.V.) for DNA isolation from filamentous fungi. DNA Isolation and purification were performed according to the suppliers' protocol, but slightly modified: protein precipitation steps 3 and 4 were repeated.

Chromosomal DNA was used as a template in a PCR reaction using primers 12207 (SEQ ID No. 8) and 11937 (SEQ ID No. 7) to amplify the insert(s) present in the expression cassette integrated into the chromosomal DNA.

Direct PCRs on transformants were performed according to an adapted version of a known protocol[4] where the mycelium obtained was subsequently treated with GlucanexJ (Novo Nordisk) at 5 mg/ml concentrations instead of the NOVOzyme.

PCR reactions contained eLONGase™ B buffer (Life Technologies, Breda, The Netherlands), dNTPs (200 $\mu$M of each), 1 $\mu$l eLONGase™ Enzyme Mix, 1-5 $\mu$l template, and 10-30 pmol of each oligo, in a final volume of 50 $\mu$l. The optimal amount of oligos was determined experimentally for each purchased batch. On average, 10 to 30 pmol was used. Reactions were performed with the following cycle conditions: 1x (2 min )94°C, 35x (1min 94°C, 1 min 55°C, 6 min 72°C), 1x (7 min 72°C). Samples were loaded on agarose gels for analyses of PCR products.

The thus obtained PCR product was subcloned in the E. *coli* pcr2.1 cloning vector (Invitrogen, according to the supplier's instructions), resulting in plasmid pGBCEA-1.

The subcloned PCR product was sequenced. The resulting nucleotide sequence of the coding region is depicted in SEQ ID NO 1 and the deduced amino acid sequence of the protein in SEQ ID NO 2. This protein has been named CEA.

Example 6: Viscometric screening of a cDNA Expression Library using the Hamilton Visco-robot.

*Viscometric measurements*

Capillary viscometers are the most commonly used type of viscometers used for the measurements of viscous liquids. In general, the liquid of interest is made to flow through a capillary tube under a known pressure difference. Then the rate of flow is measured, usually by noting the time taken for a given volume of liquid to pass a graduation mark. Other types of capillary viscometers force the liquid through a capillary at a predetermined rate of flow, and the pressure drop thereby produced across the capillary is measured in order to determine the liquids viscosity. Controlled enzymatic degradation of polymeric viscous solutions may be used to determine the enzyme activity. Providing the numeric relationship between the viscosity and the concentration of the liquid is known, kinetic parameters such as the Michaelis-Menten constant can be estimated.

I. The Detection System

(a) *The setup of the Hamilton viscorobot*

The Hamilton pipetting robot (Hamilton Workstation Microlab 2200, Hamilton Company, Reno, USA) was controlled by a software program called Eclipse (Hamilton Company). This software enables the user to direct the probe-arm to a certain position within the Hamilton workspace. A standard visco-assay Eclipse program was developed. This program works on 96-welled MTPs, where each well can be addressed individually. The syringes are used to aspirate or dispense sample liquid at an Eclipse-specified position through the needle orifice. Both the aspiration and dispension velocity (in sec/ml) as well as the aspiration and dispension volumes (in $\mu$l) were specified at will. By implementing rack definitions, multiple reagent containers can be accessed in an efficient and timesaving manner. The carry-over of sample liquid from one pipetting step to the next was avoided by instructing Eclipse to rinse the tubing with system fluid. The aspiration velocity of 10 sec/ml (sometimes 15 sec/ml) was suitable to distinguish most viscosities by looking at the peak heights. Usually 200 - 250$\mu$l of sample liquid was aspirated and dispensed.

(b) *Setup of the Hamilton*

Aspiration of sample liquid produces a pressure drop throughout the water-filled tubing. The magnitude of the pressure drop depends on both the aspiration (or plunger movement) velocity and on the viscosity of the sample liquid. The greatest pressure drop is created in the needle tip, since the radius is smallest (about 0.3 mm). Aspiration of sample liquid causes pressure changes, which are relayed from the T-junction to the pressure transducer. The transducer (Dépex B.V., de Bilt, The Netherlands) converts the magnitude of the underpressure into an electric current. The electric current is read by the Datataker once per second, and subsequently stored in the Datataker's memory in a digital format. A computer that is connected to the Datataker enables (via the Delogger software) the download of the Datataker memory-into files. They in turn can be read and analysed by common spreadsheet programs (such as Microsoft Excel). Since the pressure was measured with time, the final output can be visualised by plotting the magnitude of the underpressure (in mV) against the time (in seconds). Upon displacement of the T-junction to a position right behind the needle, the pressure signal is less dependent on the aspiration volume.

(c) Preparation of calibration curves allowed the determination of the viscosity of any liquid

According to Poisseuille's Law, the measured pressure drop should be directly proportional to the viscosity, since

$$P = (mV)F \frac{Q8\eta l}{\pi r^4} = \eta \frac{1}{k} \quad \text{where} \quad k = \frac{\pi r^4}{8lQ}$$

With this formula it possible to relate the milli-Volt underpressure output to the unknown viscosity of the sample liquid. This was done by aspirating a defined constant volume of calibration liquid (usually glycerol, as it covers a wide range of viscosities and behaves in a Newtonian manner) at various concentrations. The underpressure thus created relate to the mV output by an unknown factor F. Since the absolute viscosities of various glycerol concentrations are known, a plot of the mV underpressure output for various known glycerol concentrations against the known absolute viscosities of the same glycerol concentrations produces a straight line through the origin (since P = (mV)*F = η/k).

(d) Eclipse program used for Full-Scale Screening.

A single measuring cycle consists of the aspiration of 250μl sample and supernatant substrate mix) at an aspiration velocity of 10sec/ml. Prior to the aspiration of sample fluid, 40μl of air was aspirated to separate system from sample fluid. Once the aspiration of sample liquid was complete, it is followed by dispension of 290μl of sample and air. This measuring cycle was repeated six times, and followed by a 5ml washing step in order to clean the tubing.

II. Assay Development

(e) *Establishing the optimum substrate mixture of pectin and xylan for screening.*

Using more than one viscous substrate at a time had several advantages. One can screen for different types of enzymes at the same time. This saved a large amount of effort and time while less substrate was required. In addition to oat spelt xylan it was decided to use pectin as the second substrate. A 1:1 solution of 1% pectin and 7% oat spelt xylan seemed to be the most suitable for screening. Since there were proportionally few positive clones in the library, most peaks had an approximate height of 435mV. In case of a positive xylanase sample, all or most of the xylan was be degraded so that only 0.5% pectin was left. Hence, a positive xylanase clone produced a peak height of 280mV. In case of a pectin degrading clone, the peak height was 220mV.

(f) *Determination of the minimal xylanase activity necessary to be detected with the Hamilton viscorobot.*

The screening was carried out with very small volumes of both substrate and supernatant, since a single MTP well holds at most 360μl. Further, the more supernatant added, the more the substrate was diluted, and the more the initial viscosity is reduced. μl of 6% oat spelt xylan was dispensed into the wells of an MTP. Dilution of a series of a reference xylanase (*A. tubigensis* xylanase with an activity of 685,400 EXU/g, where 1 EXU =4.53 mole reducing sugars /min/g) were prepared and 30μl of each dilution was added to the substrate. After incubation for 24 hours at 50°C, the viscosity of each sample was measured. The lowest detectable enzyme concentration corresponds with 53.6ng/ml or 0.0367 EXU/ml. Hence, the addition of 20μl of supernatant to 300μl substrate will enable the detection of extremely low enzyme activities.

(g) *Setting up the screening for the identification of thermostable enzymes.*

In order to pick up only thermostable enzymes from the library and in order to avoid interference of host-*A.niger* enzymes activity, the plates with the clones containing the thermostable candidate enzymes were subjected to heat treatment. The system was validated using empty host strains, host strains expressing thermo-labile enzymes and host strains expressing thermostable enzymes. After growth of the strains and production of enzyme the MTP plates were heated at 72°C for 30 minutes. Subsequently 20μl of supernatant was added to 300μl of 6% oat spelt xylan. Along with negative controls (addition of 20μl of water), the plates containing the samples were sealed with sticky tape and incubated at 60°C in the oven. The high temperature may increase any thermostable enzyme activity but destroys both the background host- *A. niger* enzyme interfering activity and the non-thermostable enzyme activities expressed by the library. After 20 hours of incubation, no notable peak height- decrease was found for the thermo-labile xylanase clones, indicating that host-xylanase activity has been thoroughly inactivated. In addition clones of the thermolabile xylanase enzyme xynB of *Aspergillus niger* were included as a control. In this case no residual activity could be detected, while on the other hand the heat resistant xylanase which was included as a control was still active. Heat inactivation for 30 minutes at 72°C and sample incubation times of 24 hours or less enabled us to specifically detect thermostable *T. emersonii* xylanases.

III. Screening of the cDNA expression library using the Hamilton Visco-robot

(h) *Replication of the cDNA expression library and expression of the library.*

One replication cycle involves the two-fold inoculation of both selective medium (SM) (containing per litre: 0.52g KCl, 1.52g $K_2HPO_4$, 0.52g $MgSO_4$, 20g glucose, 1g acetamide, 0.1M MES buffer; 15g agar 1ml of trace element solution [trace elements solution (per litre): 2.2g $ZnSO_4/7H_2O$, 1.1g $H_3BO_3$, 0.5g$FeSO_4$ /$7H_2O$, 0.17g $CoCl_2/6H_2O$, 0.16g $CuSO_4/5H_2O$, 0.15g $NaMoO_4/2H_2O$, 5.0g EDTA, pH 6.5] pH 5.5) and liquid growth medium (GM) (containing, per litre, 70g glucose, 25g casein hydrolysate, 12.5g yeast extract, 1g $KH_2PO_4$, 0.5g $K_2SO_4$, 2g $MgSO_4$, 0.03g $ZnCl_2$, 0.02g $CaCl_2$, 0.01g $MnSO_4$, 0.3g $FeSO_4$, pH 5.6). The library was stored in standard MTPs, where sporulated *recombinant A. niger* colonies grew in each well on SM in the presence of 10% glycerol. During storage, these plates (master plates, MPs) were kept in a frozen state (-80°C). Prior to the replication of the MPs, they were defrosted for one hour in a sterilised fume cupboard to avoid microbial contamination. Replication of the MPs was performed with the PBA Flexys-Colony Replicator. 5 days-old grown Master Plates were used as replication template and replica plated in 96-welled MTPs filled with liquid growth medium (GM). The freshly inoculated SM agar plates were placed in an incubator at 32°C and subsequently stored at -80°C after addition of 150µl of 10% glycerol per well. The GM production plates were incubated in a water-saturated Tomtec Quadrastor Shaker 96000 (32°C). The plates showed growing mycelium after 2-3 days. The GM plates were incubated for 6 days.

(i) *Sampling of the supernatants after the growth is complete.*

After 6 days of growth, the remaining GM liquid growth medium (containing extracellular expression products) was extracted and transferred into fresh MTPs. The supernatants were transferred into fresh MTPs using a 4-channel Tecan pipetting robot. The average volume of supernatant recovered was between 120 and 140µl. These supernatant plates were frozen and were subsequently assayed for xylanase activity.

(j) *Preparations for the full-scale screening.*

The supernatant plates from the expression library were defrosted and placed in a closed water bath at 72°C for 35-40 minutes. Using a 12-channel pipette and commercial pre-prepared pipette tips in disposable MTP-format racks (supplied by Eppendorf), 20µl of each supernatant was transferred to the substrate-mixture containing MTP which had been heated to 60°C before adding the supernatants. The substrate mixture consisted of a 1:1 mixture of 1% pectin (see (k) below) and 7% oat spelt xylan (see (1) below), with a final pH of 4.12. The assay MTPs were filled with 310ml of substrate/well. Mixing of supernatants with the substrate mixture was achieved by stirring the pipette-tips in the sample wells directly after dispensing the supernatants. Subsequently the plates were placed in a 60°C oven for an incubation period of 18-24 hours. After the incubation, the MTPs were allowed to cool down and screened for a viscosity decrease using the Hamilton visco-robot (Hamilton Company, Reno, USA).

(k) *Preparation of 1% pectin, pH 4.1*

A citric acid-phosphate buffer (Mc Ilvaine buffer) was prepared by adding 0.2M $Na_2HPO_4$ solution to 250 ml of 0.1M citric acid solution until a pH of 5.5 was obtained. This was made up to one litre with dstilled water, and the pH was readjusted if necessary. A 0.5% pectin solution was made by slowly adding 0.5g of highly methylated pectin (type Ruban Brun) to a flask containing 50ml of the above McIlvaine buffer and 25ml distilled water at 60°C. Vigorous stirring ensured that the pectin dissolved well. This was made up to 100ml, and the pH checked again. The pectin used here lowered the pH of the solution, so under these conditions the solution had a pH of 5.1. If the pectin solution was cloudy, it was useful to centrifuge the solution at 15g for 15 minutes in order to remove any undissolved particles.

(I) *Preparation of 7% alkaline-treated oat spelt xylan, pH 4.1.*

20ml of 2M NaOH was heated up to 60°C in a 150m1 beaker. In a separate beaker 7g of oat spelt xylan (from Sigma company) was added to 20m1 of water, so that a bright brown, doughy mass formed. If necessary, more water was added , but no more than 30 ml altogether. Using a steel spoon, this water-oat spelt xylan mass was slowly added to the hot NaOH solution. A powerful stirring device was required to dissolve the xylan in the hydroxide solution, thereby keeping the temperature at a steady 60°C. Once all the xylan had dissolved, the solution turned dark-brown and resembled a very viscous, clear syrup. Then the rest of the water was added so that an overall amount of 50ml had been added. The solution was allowed to cool down and 4N HCl added until the desired pH was achieved (usually pH 4.1-4.2). The solution was made up to 100ml and the pH readjusted if necessary. Centrifugation at 20,000g for 15 minutes at 10°C produced a clear yellowish supernatant (whose viscosity depended on the initial amount of oat spelt xylan added).

### (m) *The screening of the library*

The outcome of the screening of the *Talaromyces emersonii* library cloned into *A. niger* was 119 graphs corresponding with the 119 MTPs tested. Each graph showed the viscosity of the 96 wells represented as 96 peaks which measure the underpressure per well in mV. The graphs were analyzed for low peaks, which indicated reduced viscosity. Peaks that are lower than the average peak height were selected for retesting. Some plates produced very little variation, so that selection of putative positive clones for retesting was easy. Other plates showed a great amount of variation, hence often more than or 6 putative clones were selected for retesting. To have a positive control, after the incubation 10μl of an endoxylanase was added to a random plate at a fixed position. From those positive controls, it was found that if there is xylanase activity, we should expect a peak height between 240 and 280mV.
The plates of the *Talaromyces* library which were tested also contained five confirmed thermostable xylanase clones which were found before using a dye detection assay based on solubilisation of dyes which were chemically attached to the insoluble polymeric substrate. Without exception, those five clones were independently found with the viscometric assay already in the primary screening round. All peaks for the known clones were on a level much lower than the rest of the peaks (at 250mV). Overall, 118 culture wells were selected for retesting, excluding those with previously found xylanase clones.

### (n) *Retesting of 118 putative clones*

Retesting was based on the viscometric assay accordirig to the principle used with the full-scale screening. This time, however, a greater assay volume was utilised, since a clearer distinction between thermostable enzyme activity and irregular low peaks could be made. Two large MTPs (2ml/well) were filled with 1.2ml of 9% oat spelt xylan. To the first two wells of each row 50μl of water was added (negative control). To the last well of each row, 50μl of an reference endoxylanase solution was added in order to have a positive control, too. Wells 3 to 11 of each row were used for the retesting of putative xylanase clones (addition of 50μl supernatant). After mixing and incubation for 24 hours at 60°C, the viscosities were measured with a specifically designed Eclipse program (Aspiration volume: 800μl, Aspiration velocity: 10sec/ml).
Several positive clones were identified since their peaks were exactly on the same level as the positive controls, indicating complete degradation of xylan. In total thirteen putative strains were identified in the viscoscreen retest. The cDNA inserts were directly cloned in the pCR2.1 vector via PCR amplification executed on chromosomal DNA. From 12 library strains sixteen cDNA inserts were obtained, using pwo-polymerase and primer set 2 (11937/12207, SEQ ID Nos. 7 and 8). The orientation of the cDNA insert in the pCR2.1 vector was determined by XhoI digestion, which was located in the vector and at the 3' of the cDNA. DNA sequence analysis was executed on the 5' end of the cDNA inserts.
The retesting for possible pectin degrading enzymes was performed in a similar fashion as for the xylanase, except that 1% pectin was used instead of 9% oat spelt xylan. Only ten possible clones were retested, which were those that produced very low peaks in the primary screening round but did not show any activity during the xylanase retesting. Reproducibly one clone produced a pectin-degrading enzyme. The measured pressure value of 200mV corresponds to the viscosity of pure water. Since the pectin used was not fully methylated, it might have been degraded by a thermostable polygalacturonase, pectate lyase or pectin lyase.

### (o) *Benefits of Viscoscreen*

The development of a viscometric screening method to identify thermostable enzymes was facilitated by three key factors. Firstly, the possibility of thermo-inactivation of native host-xylanase activity allowed a much faster development of appropriate reaction conditions. Secondly, the fact that when clones with known thermo-labile enzymes were tested the thermoinactivation destroyed all activity so that only the more thermostable enzymes were picked up. In the third place the elevated temperatures increased the enzymatic activities so that the screen became even more sensitive. On Oat Spelt Xylan (Sigma) eleven clones were identified which were able to reduce viscosity significantly and on pectin one definite pectin-degrading clone was identified. Additionally, five thermostable xylanase clones which were previously identified were independently re-discovered.

Example 7: Characterization of first *Talaromyces emersonii* thermostable β-glucanase (CEA) from *A.niger* transformant

*Activity assays and definitions*

*Cellulase PAHBAH Unit (CPU) definition*

One unit of cellulase activity is defined as the amount of enzyme required to release one $\mu$mol reducing sugars produced per minute at pH 5.0 and 60°C at a substrate concentration of 5% Carboxy Methyl Cellulose (CMC), using a calibration curve of glucose.

Enzyme activity according to the CPU method was measured by detecting reducing sugars using 4-hydroxybenzoic acid hydrazide (PAHBAH). The assay is based on Lever, M., Powell, J.C., Killip, M., Small, C.W. (1973) J. Lab. Clin. Med. 82, 649-655 with some modifications. The modification is to the PAHBAH reagent as follows : 0.05 M trisodium citrate, 0.1 M $Na_2SO_3$, 0.02M $CaCl_2$, 0.5M NaOH and 0.1M p-hydroxybenzoic acid hydrazide (PAHBAH). Final pH was 12. The reagent containing PAHBAH in alkaline solution, stored at room temperature, should be used within one day. Glucose was used as the reference reducing sugar (calibration curve). For the calibration curve of this assay, the final concentrations of glucose were between 0-300 mM. The CPU activity was assayed by mixing 100 $\mu$l of enzyme solution with 400 $\mu$l of 5% CMC in 0.1 M sodium acetate buffer (pH 5.0). Eppendorf cups with the substrate (CMC) were preincubated for 5 minutes at 60°C. The reaction was started by adding the enzyme solution. After 15 minutes the reaction was stopped by adding 1.0 ml PAHBAH-reagent. The Eppendorf cups were heated for 5 minutes at 100°C and then cooled on ice. Samples were centrifuged at the appropriate speed in order to spin down any solid materials (1 minute at full speed in a Beckman Microfuge E). The absorbance was measured at 420 nm. A blank was prepared by adding 100 $\mu$l 0.1M sodium acetate buffer instead of enzyme solution.

*Beta Glucanase Unit (BGU) definition*

Beta Glucanase Unit is the activity required to liberate 0.258 $\mu$mol reducing sugars (measured as glucose equivalents) per minute at pH 3.5 and 40°C, at a substrate concentration of 0.5% β-glucan from barley.

So in addition to the above determination of cellulase (CPU) activity, a more specific assay was carried out for detecting ß-glucanase activity. The principle of the assay is the velocity at which the viscosity decreases of a solution of barley ß-glucan medium viscosity (Megazyme, Australia, 2/11 Ponderosa Parade, Warriewood NSW 2101) upon addition of a certain amount of enzyme. Barley β-glucan, medium viscosity (Megazyme Australia), was dissolved in 0.425M sodium citrate buffer pH 3.5 to a concentration of 6.25mg/ml. The substrate was incubated at 40°C for 10 minutes. Subsequently a small amount of enzyme (in the range 0.005-0.062 Units/ml) was added and the reaction allowed to proceed. At 60 minutes reaction time the viscosity of the sample was determined relative to a reference sample which was incubated with a standard endo-glucanase of known enzymatic activity. Absolute activities for the standard were determined by a reducing sugar method using Fe-III-hexacyanide and 4.76 mg/ml barley ß-glucan as initial substrate concentration.

*Definition of Endo Xylanase Unit (EXU)*

The unit of xylanase activity (EXU) is defined as the amount of enzyme (endoI endo-1,4-β-xylanase from *Asp. niger,* as decribed in EP-A-0,463,706 (Gist-brocades B.V.)) that liberates 4.53$\mu$mol reducing sugars (measured as xylose equivalents) per minute under assay conditions. The assay conditions comprise: 5mg/ml arabinoxylan from wheat flour (Megazyme, Australia, 2/11 Ponderosa Parade, Warriewood NSW 2101) in 100mM sodium citrate buffer (pH 3.5), temperature 40°C, at a reaction time of 60 minutes. Reactions were stopped by adding 1M NaOH. Detection was done colorimetrically at 420nm after incubating the samples with Fe-III-hexacyanide for 15 minutes in boiling water. The hexacyanoferrate reagent was made up by dissolving 1.17g KFe(CN) and 19.5g anhydrous sodium carbonate in 1 litre of water.

In addition to the above absolute determination of xylanase activity, a relative method was used that followed the decrease in viscosity of a solution of wheat arabinoxylan (Megazyme, Australia, 2/11 Ponderosa Parade, Warriewood NSW 2101) upon addition of a certain amount of enzyme. Wheat arabinoxylan was dissolved in 0.425M sodium citrate buffer (pH 3.5) to a concentration of 8.3mg/ml. The substrate was incubated at 55°C for 10 minutes. Subsequently a small amount of enzyme (in the range 0.01-0.05 Units/ml) was added and the reaction allowed to proceed. After 60 minutes reaction time the viscosity of the sample was determined relative to a reference which was incubated with a *Aspergillus niger* endo-xylanase standard of known EXU activity (EP-A-0,463,706). Absolute activities in EXU for the standard were determined by reducing sugar method using Fe-III-hexacyanide as described above. Viscosity was determined manually using a Haake falling ball viscosity apparatus.

*Definition of Cellulase Unit (CXU)*

The unit of beta-glucanase activity (BGU) is defined as the amount of cellulase that hydrolyses in one hour a number of glycosidic bonds equivalent to the production of 0.5 mg glucose under the conditions of the assay. The assay conditions comprise: 9mg/ml carboxymethylcellulose in 5mM sodium acetate buffer (pH=4.6), temperature 37°C. Liberated gluxose was determined by reducing suger method using the DNS reagent.

In addition to the above absolute determination of cellulase activity, a relative method was used that followed the decrease in viscosity of a solution of carboxymethylcellulose upon addition of a certain amount of enzyme. Carboxymethylcellulose was dissolved in 5mM sodium citrate buffer (pH=4.6) to a concentration that depends on the viscosity of the batch. The substrate was incubated at 37°C for 10 minutes. Subsequently a small amount of enzyme was added and the reaction allowed to proceed. After 60 minutes reaction time the viscosity of the sample was determined relative to a reference which was incubated with a standard of known CXU activity.

*Activity wrt pH*

The *T.emersonii* ß-glucanase was produced by the appropriate *A.niger* transformant(s) in shake flasks as described in Example 3 after growth mycelium was removed by filtration. The filtrate was used for this experiment. The activity of the filtrate was measured at different pH values at a fixed temperature of 60°C. The activity was measured according to CPU method.-Instead of using the fixed pH of pH 5.0, the CMC substrate was diluted with a citrate buffer of the appropriate pH in order to obtain the pH of measurement. The experiment was repeated twice and the results are shown below in Table 1. The optimal pH of the enzyme was found to be between pH 4.5 and 5.0, at about pH 4.8.

Table 1: pH profile at 60°C

| pH | activity (μM glucose/15 minutes) at 60°C (duplicate measurements) | |
| --- | --- | --- |
| 3 | 27.2 | 27.7 |
| 3.5 | 66.6 | 64.2 |
| 4 | 109.3 | 102.6 |
| 4.5 | 133.8 | 120.6 |
| 5 | 121.3 | 135.5 |
| 5.5 | 116.4 | 107.4 |
| 6 | 68.7 | 69.3 |

*Activity wrt temperature*

The activity of this β-glucanase was then measured at different temperatures. Activity measurements were carried out according to the CPU method at pH 4.0. Instead of incubating at a fixed temperature of 60°C, the incubations were performed at various temperatures. The experiment was performed twice and the results are shown below in Table 2. The temperature optimum was found to be between 80°C and 85°C. The $T_{opt}$ seems to be at about 84°C (although it can be 82°C, 83°C or 85°C, depending on how the lines are drawn and interpolated between data points).

Table 2: Temperature profile at pH 4

| Temperature (°C) | activity (μM glucose/15 minutes): (duplicate measurements) | |
| --- | --- | --- |
| 60 | 87.8 | 102.1 |
| 70 | 156.4 | 154.6 |
| 80 | 208.5 | 213.1 |
| 90 | 185.4 | 176.4 |

*Activity wrt other enzymes*

In addition the activity at 30-60°C was measured using the BGU method for the β-glucanase and compared with a commercially available *T.reesei* cell free broth having cellulase and glucanase activity as a reference. *T.reesei* cell free broth consisted of a mixture of different enzymes among which are one or more β-glucanases but where these activities have not been characterized. For comparing the activities of *T.emersonii* β-glucanase of the invention and *T.reesei* cellulases/glucanases, the enzymes were dosed at about equal activity at 40°C. The activity of *T. emersonii* β-glucanase A at 40°C was set to one and so other activities are relative to this. The results are shown below in Table 3.

Table 3: relative activity wrt T. *reesei* enzymes

| Temperature (°C) | Relative Activity: β-glucanase (CEA) | Relative Activity: *T. reesei* Cellulases/glucanases |
|---|---|---|
| 30 | 0.50 | 0.44 |
| 40 | 1.00 | 1.13 |
| 50 | 1.57 | 1.32 |
| 60 | 2.40 | 1.59 |

At temperatures above 40°C the *T.reesei* cellulase/glucanase activity started to level off when compared to the *T. emersonii* β-glucanase. Above 40°C the activitys diverged, CEA being more active. Also, while relative activity was higher at elevated temperatures, at moderate temperatures activity was maintained relative to the *T. reesei* mixture. This illustrates the wide temperature range over which the β-glucanase is active.

*Purification and Specific Activity*

Purification of the *T.emersonii* β-glucanase started from the filtrate. About 25 ml cell free broth was desalted with a PD 10 column and placed on a resource Q 6 ml anion exchange column which was equilibrated in 10 mM sodium acetate buffer (pH 5.0). Elution was carried out with a linear gradient from 10-300 mM sodium acetate buffer (pH 5.0). (Linear gradient A to B; flow rate: 6 ml/min ; run time: 54 min; wavelength monitor: 280 nm, 254 nm, 214 nm). Active fractions were collected (20 ml), concentrated with Microsep concentrators (3.5 ml, 10 K) and washed twice with 0.1 M sodium phosphate buffer (pH 5.0). The purity of the fractions was analyzed by HPL-SEC (size exclusion chromatography) and SDS-PAGE.

*Specific activity measurements*

The calculated molar extinction coefficient of the β-glucanase at 280 nm is 81550 $M^{-1}.cm^{-1}$. The protein concentration of the purified enzyme was derived from E280 measurements using a specific absorption of E280$^{1cm}$=2.33 for 1mg/ml. The specific activity of the purified sample was determined in BGU (substrate: barley beta-glucan) and was 1027 BGU/mg. Using the CPU method the specific activity was 628 units/mg. As the activity according to the viscosimetric method yielded a high number in comparison with the reducing sugar method, it was concluded that the *T.emersonii* β-glucanase exhibits significant endo-glucanase activity. A typical exo-glucanase would perform very well in a reducing sugar assay while performing very badly in the viscosimetric assay.

**Iso-Electric Point**

IEF-PAGE was performed as follows. Equipment was the Phast system (Pharmacia Biotech), IEF 3-9 PhastGel (Pharmacia Biotech). Gels were run and stained (Coomassie) according to standard Phast system methods. The Iso-Electric Point was determined on PhastGel IEF3-9 and turned out to be 3.3.

**Molecular Weight Determination**

SDS-PAGE was performed as follows. Equipment was again the Phast system (Pharmacia Biotech); 12.5% homogeneous gels (Pharmacia Biotech); SDS-buffer strips (Pharmacia Biotech). Sample treatment: one volume (5 samples) buffer (500mM Tris-HCl, pH 6.8, 10% SDS, 0.1% Bromo-phdnol blue) was mixed with 4 volumes of sample and boiled for. 3 minutes. Gels were run and stained (Coomassie) according to standard Phast system methods. HPLC-size exclusion chromatography was carried out using Column: TSK G3000SW, cat. no. 05103 (TosoHaas). Method: eluents were: 0.1 M Sodium phosphate buffer pH 7.0, Flow rate: 1ml/min, Run time: 30 min, Wavelength monitor: 280 nm. Deglycosylation of the enzyme: mix 5μl purified enzyme (7 mg/ml) with 20 μl 0.5% SDS and 25 μl 1% ß-mercaptoethanol. This mixture was boiled for 4 minutes. After cooling down, 20 μl N-glycosidase F (500 U/ml) and 20 μl 3% Triton X-100 in 1 M sodium phosphate buffer pH 7.0 were added. This was incubated overnight and the deglycosylation analysed with SDS-PAGE.

The molecular weight determined on SDS-PAGE gel and HP-SEC turned out to be 43 kDa. After deglycosylation on SDS-PAGE a straight band was seen at 37 kDa.

*Thermostability*

T50 thermostability was determined with the *T.reesei* cellulases/glucanases as a reference. T50 is the temperature at which, after 20 minutes of incubation, 50% of the activity is left. Table 4 shows the differences in T50 thermostability between the purified β-glucanase from *T. emersonii* and the *T.reesei* cellulase/glucanases mixture used earlier in this Example (initial activity was set to one: the enzymes were incubated for 20 minutes and after cooling activity was measured using the CPU method).. Each experiment was repeated twice and so Table 4 shows duplicate measurements. The T50 thermostability of the purified β-glucanase lies around 93.4°C and the T50 thermostability of *T.reesei* cellulase / glucanases around 64.9°C.

Table 4: thermostability wrt *T. reesei* enzymes

| Temperature (°C) | Residual Activity (%) *T. reesei* cellulases/ glucanases | | Residual Activity (%) β-glucanase (CEA) | |
|---|---|---|---|---|
| 40 | 97 | 98 | | |
| 50 | 96 | 100 | 100 | 99 |
| 60 | 85 | 83 | 100 | 99 |
| 70 | 14 | 14 | 98 | 95 |
| 75 | 0 | 0 | 93 | 96 |
| 80 | 0 | 0 | 88 | 90 |
| 85 | 0 | 0 | 89 | 94 |
| 90 | 0 | 0 | 65 | 64 |
| 110 | | | 0 | 0 |

Example 8: Activity Measurements

The two library strains which were identified in the viscoscreen in Example 6, using Oat Spelt Xylan as a substrate, were fermented in shake flasks using the GM medium (the original strains were labelled AD009.21 and AD011.31). In addition the strain labelled AD021.B6 (CEA) was included which was identified in Examples 4 and 5 to express β-glucanase activity. Several assays were performed and the results are shown below. Surprisingly strains AD009.21 and AD011.31 showed little xylanase activity. Instead it seems that AD009.21 and AD011.31 have cellulase activity even though the clones were identified in the viscoscreening using oat spelt xylan. In particular p D009.21 is very active on barley β-glucan

Table 5: Shake flask fermentation of cellulase strains; Xylanase (EXU/ml), ß-glucanase (BGU/ml) and cellulase (CXU/ml) activity.

| Strain (and glucanase designation) | EXU/ml | BGU/ml | CXU/ml |
|---|---|---|---|
| AD021.B6 (CEA) | <10 | 160 | 524 |
| AD009.21 (CEB) | <10 | 884 | 254 |
| AD011.31 (CEC) | <10 | <10 | 7 |

The detection limits were set to below 10 for the EXU and BGU assays, and 5 for the CXU assay.

The DNA analysis on strains AD 009.21 and AD 011.31 (as described earlier in Example 6) showed two new β-glucanases, called CEB and CEC. The nucleotide sequences of the coding regions are SEQ ID Nos. 3 and 5 and corresponding amino acid sequences are SEQ ID Nos. 4 and 6, respectively.

The CEB strain shows high β-glucanase activity. The CEA strain shows a relative high cellulase activity in comparison with the CEB strain. The ratio between the β-glucanase activity and the cellulase activity is different for CEA and CEB. The CEC strain showed cellulase activity but its β-glucanase or xlyanase activity was below limits of detection in these assays. However, homology studies strongly suggest β-glucanase activity, and it is thought that this may be much higher at different pH's.

Using more than one substrate for the screening is feasible. Surprisingly it turned out that instead of two substrates at a time, three diffent substrates could be used in the viscoscreen. One of the substrates was a glucose polymer impurity, likely glucan or cellulose material. It was shown that the use of oat spelt xylan, pectin and cellulose like material resulted in the identification of three types of enzymes, xylanases, pectinases and cellulases in one round of screening. The viscometric assay holds one other great advantage with respect to the common dye detection assay. The dye detection assay bases on dyes chemically linked to a substrate. This kind of substrate is commercially available for only a few compounds. The viscometric assay, however, can work with any type of viscous substrate, even natural ones that do not require any chemical pre-treatment. This aspect is of importance as, from a commercial and industrial point of view, one can screen for enzyme activities on substrates whose viscosity-decrease {i.e. fruit juices) or viscosity-increase (i.e. dairy products) is of commercial importance.

Example 9: Baking performance of the *Talaromyces emersonii* endoglucanase (CEC)

Preparation of tin bread in a standard baking process was performed by mixing 3500g wheat flour (a mix of 80% Kolibri and 20% Ibis wheat flours (Meneba, Holland) at about 21°C), 77g compressed (Konings) yeast, 70g salt, 25ppm ascorbic acid, 10ppm fungal α-amylase Fermizyme™$P_{200}$, (DSM N.V., Bakery Ingredients, Delft, The Netherlands) and different quantities of the endoglucanase CEC enzyme (from 4 different clones, identified by their AD number) and 2030mL water (8-15°C) in a spiral mixer (Hobart) for 2 minutes (at speed 1) and for about 6 minutes (at speed 2) to put in 125Wh (Watt-hours) of energy. The dough temperature was 28°C. Machineability of the dough was analysed by hand by a qualified baker.

Directly after mixing the dough was divided into 6 pieces each of 875g, rounded and proofed for 35 minutes in a proofing cabinet at 34°C and 85% RH (relative humidity). At the end of this period the doughs were shaped and panned and given a final proof of 75 minutes in a proofing cabinet at 38°C and 87% RH. Afterwards the fully proofed doughs were baked in an electric oven at 210°C for 30 minutes. After cooling to room temperature the volumes of the loaves of bread were determined by the rape seed displacement method. After 16-24 hours storage in sealed polyethylene bags at room temperature the crumb quality was assessed by a qualified baker. Four library strains which were identified to express CEC were fermented in shake flasks and tested in this baking test. The results are shown in the Tables below.

| Dosage level of CEC AD 011.31 (CXU/kg flour) | Loaf volume | | Dough handling | Crumb quality (scale 0-7) |
|---|---|---|---|---|
| | (mL) | (%) | | |
| 0 | 4105 | 100 | easy, not sticky | 6 |
| 56 | 4357 | 106 | easier, not sticky | 6 |
| 189 | 4289 | 104 | easier, some stickiness | 6.5 |

| Dosage level of CEC AD 046b.21 (CXU/kg flour) | Loaf volume | | Dough handling | Crumb quality (scale 0-7) |
|---|---|---|---|---|
| | (mL) | (%) | | |
| 0 | 4105 | 100 | easy, not sticky | 6 |
| 56 | 4427 | 108 | easier, not sticky | 6 |
| 189 | 4419 | 108 | easier, some stickiness | 6 |

| Dosage level of CEC AD 050.13 (CXU/kg flour) | Loaf volume | | Dough handling | Crumb quality (scale 0-7) |
|---|---|---|---|---|
| | (mL) | (%) | | |
| 0 | 4105 | 100 | easy, not sticky | 6 |
| 56 | 4432 | 108 | easier, not sticky | 5.5 |
| 189 | 4622 | 113 | easier, not sticky | 6 |

| Dosage level of CEC AD 078.50 (CXU/kg flour) | Loaf volume (mL) | (%) | Dough handling | Crumb quality (scale 0-7) |
|---|---|---|---|---|
| 0 | 4105 | 100 | easy, not sticky | 6 |
| 56 | 4450 | 108 | easier, not sticky | 5.5 |
| 189 | 4553 | 111 | easier, some stickiness | 6 |

The quality of the doughs was very good. At the higher dosing for endoglucanase CEC there was a little stickiness experienced during handling of the dough. However this little stickiness did not influence the dough's machineability. All doughs containing endoglucanase CEC were very supple and easy to handle.

From these baking results it was concluded that endoglucanase CEC is very effective in improving bread quality, both in terms of loaf volume and in terms of crumb quality. Despite the large volumes of the loaves the crumb structure was still very regular and fine.

Example 10: Comparison of baking performance of the *Talaromyces emersonii* enzyme (CEC) with endoxylanase from *Asp. niger*

The baking performance of CEC was compared in Dutch tin bread production with a currently used fungal endoglucanase from *Aspergillus niger*. This *A. niger* endoglucanase was supplied in its pure commercially available form, i.e. Fermizyme™ HS$_{2000}$.

The exact procedure of Example 9 was repeated except different quantities of either the endoglucanase CEC or Fermizyme™ HS$_{2000}$ were used.

| Enzyme | Dosage level (CXU/kg flour) | Loaf volume (mL) | (%) | Dough handling | Crumb quality (scale 0-7) |
|---|---|---|---|---|---|
| endoglucanase CEC 050.13 | 0 | 4105 | 100 | Good, not sticky | 6 |
| | 56 | 4432 | 108 | Supple, not sticky | 5.5 |
| | 189 | 4622 | 113 | Supple, not sticky | 6 |

| Fermizyme™ HS$_{2000}$ | Dosage level (EXU/kg flour) | Loaf volume (mL) | (%) | Dough handling | Crumb quality (scale 0-7) |
|---|---|---|---|---|---|
| | 0 | 4123 | 100 | Good, not sticky | 6 |
| | 527 | 4361 | 106 | Supple, not sticky | 6 |
| | 1056 | 4535 | 110 | Supple, bit sticky | 7 |

From the results it is clear that the endoglucanase CEC improved loaf volume to a larger extent than obtained by introducing Fermizyme™ HS$_{2000}$. Moreover, less endoglucanase units/kg flour were needed to reach a certain level in loaf volume when CEC was used instead of Fermizyme™ HS$_{2000}$.

Example 11

A comparison of some of the molecular and biochemical properties of the three glucases is provided in the Tables below.

| Glucanase | MW (after deglycosylation) | Length (amino acid residues) | Activity | Family No.* | Optimum pH | pI | Optimum Temperature |
|---|---|---|---|---|---|---|---|
| CEA | 43kDa (37 kDa) | 335 | 3.2.1.4 (endoglucanase) | 5 (3D structure: $\alpha$8ß8 TIM barrel) | 4.8 | 3.3 (4.5[+]) | 85°C |
| 2. CEB | 44kDa[+] | 414 | 32.1.4 (endoglucanase) | 7 (3D structure: B-jelly roll) | | 42 | >75°C |
| CEC | 23kDa[+] | 222 | 3.2.1.4 (endoglucanase) | 45 (3D structure: mixed ß-barrel) | | 4.0 | >75°C |

* Based on glycoside hydrolase (CAZy) classification
+ Predicted from amino acid sequence

| Glucanase/ Cellulase | Family | Known Activities | Known Activites | Conclusion |
|---|---|---|---|---|
| CEA | 5 | EC 3.2.1.4<br>EC 3.2.1.75<br>EC 3.2.1.58<br>EC 3.2.1.78<br>EC 3.2.1.123 | cellulase<br>endo 1,6 glucanase<br>exo 1,3 glucanase<br>mannanase<br>endoglycoceramidase | BGU/CXU activity<br><br>Considered unlikely |
| CEB | 7 | EC 3.2.1.4<br>EC 3.2.1.91 | cellulase<br>cellobiohydrolase | BGU/CXU activity<br>considered unlikely |
| CEC | 45 | EC 3.2.1.4 | cellulase | BGU/CXU activity but at different pH from that tested |

| Glucanase/ Cellulase | Family | 3D | Nucleophile/ Proton Donor | Mechanism |
|---|---|---|---|---|
| CEA | 5 | TIM 8 | glu/glu | retaining |
| CEN | 7 | jelly roll | glu/glu | retaining |
| CEC | 45 | mixed barrel | asp/asp | inverting |
| | 16 | jelly roll | glu/glu | retaining |

CEB, CEC are all cellulases that exhibit EC 3.2.1.4 activity. Many cellulases can also hydrolyse 1,4 bonds in barley -glucan which makes them particularly useful for certain applications such as e.g. eliminating anti-nutritional factors in feed. As activity was measured via the viscosity assay it is likely that the cellulase exhibited endo activity. It cannot be excluded however that also 1,3 linkages are hydrolysed. Hence, on the observations, EC 3.2.1.73, 3.2.1.39 and 3.2.1.6 activities cannot be ruled out even though these activities are found in family 16: the characteristcs of families 7 and 16 seem rather similar.

Purel as explanation, cellulases (EC 3.2.1.4) are usually able to catalyse the endo hydrolysis of 1,4-D-glucosidic linkage in cellulose. The systematic name of cellulases is 1,4-(2,3;1,4)-D-glucan 4-glucanohydrolase. Endo-1,4-D-glucanase and endoglucanase D are synonyms of 1,4-(1,3;1,4)-D-glucan 4-glucanohydrolase. Cellulase may also hydrolyse 1,4-linkages in -D-glucans containing also 1,3 linkages, which makes them particularly useful for certain applications such as e.g. eliminating anti-nutritional factors in feed. Such enzymes which exhibit endo-glucanase activity are referred to in general as glucanases. In addition hydrolyis of glucan can be achived by lichenase (1,3-1,4-D-glucan glucanohydrolase (EC 3.2.1.73)) and endo-1,3(4)-glucanase (1,3-(1,3;1,4)-D-glucan 3(4) glucanohydrolase (EC 3.2.1.6)).

A review of cellulase activities is shown in the table below.

| EC number | Activity | Systematic Name | Recommended | Synonym |
|---|---|---|---|---|
| 3.2.1.4 | Endo Hydrolysis of 1,4-β-D-glucosidic linkage in cellulose. Will also hydrolyse 1,4-linkages in β-D-glucans also containing 1,3 linkages. | 1,4-(1,3;1,4)-β-D-glucan 4-glucanohydrolase | cellulase | Endo-1,4-β-D-glucanase endoglucanase D |

(continued)

| EC number | Activity | Systematic Name | Recommended | Synonym |
|---|---|---|---|---|
| 3.2.1.73 | Hydrolysis 1,4-β-D-glycosidic linkages in β-D-glucans containing 1,3 and 1,4 bonds. Acts on lichenin and cereal β-D-glucans, but not on β-D-glucans containing only 1,3 or only 1,4 bonds. | 1,3-1,4-β-D-glucan glucanohydrolase | Lichenase | |
| 3.2.1.39 | Hydrolysis 1,3 β-D-glucosidic linkages in 1,3 P-D-glucans. Limited activity on mixed (1,3-1,4) β-D-glucans | 1,3-β-D-glucan glucanohydrolase | Glucan endo-1,3-β-glucosidase | endo 1,3 β-D-glucanase β 1,3 glucanase Oligo 1,3 glucosidase |
| 3.2.1.58 | Succesive hydrolysis of β-D-glucose units from the non-reducing ends of 1,3-β-D-glucans, releasing α-glucose | 1,3-β-D-glucan glucohydrolase | glucan 1,3-β-glucosidase | exo β-1,3-glucanase |
| 3.2.1.75 | Random hydrolysis of 1,6 linkages in 1,6-β-D-glucosides. acts on lutean, pustulan, 1,6-oligo-β-D-glucosides | 1,6-β-D-glucan glucanohydrolase | glucan endo-1,6-β-glucosidase | endo-β-1,6-glucanase β-1,6-glucan hydrolase |
| 3.2.1.6 | Endohydrolysis of 1,3 or 1,4 linkages in β-D-glucans when the glucose residue whose reducing group is involved in the linkage to be hydrolysed is itself substituted at C-3. Substrates include cereal D-glucans, laminarin, lichenin | 1,3-(1,3;1,4)-β-D-glucan 3(4) glucanohydrolase | endo-1,3(4)-β-glucanase | β-1,3-glucanase beta-1,3-1,4-glucanase endo-β,1,3 (4)-glucanase |
| 3.2.1.91 | Hydrolysis of 1,4-β-D-glucosidic linkages in cellulose and cellotetraose, releasing cellobiose from the non-reducing end | 1,4-D-glucan cellobiohydrolase | cellulose 1,4-β-exoglucanase cellobiosidase | cellobiohydrolase exo-cellobiohydrolase |

<u>REFERENCES</u>

1. Sambrook et al. (1989) "Molecular Cloning: A laboratory manual", 2nd Edition, Cold Spring Harbor Laboratories, Cold Spring Harbor, New York

2. Innis et al. (1990) "PCR protocols, a guide to methods and applications" Academic Press, San Diego.

3. WO-A-99/32617

4. van Zeijl, C. et al. (1998) J. of BiotechnoL 59: 221-224

5. Devereux et al (1984) Nucleic Acids Research 12, p387-395

6. Altschul S. F. (1993) J Mol Evol 36:290-300

7. Altschul, S, F et al (1990) J Mol Biol 215:403-10

8. Henikoff and Henikoff (1992) Proc. Natl. Acad Sci. USA 89: 10915-10919)

9. Karlin and Altschul (1993) Proc. Natl. Acad Sci. USA 90: 5873-5787

10. Cunningham and Wells, Science, 244, 1081-1085, 1989

11. de Vos et al. (Science, 255, 306-312, 1992)

12. Smith et al. (J. Mol. Biol., 224, 899-904, 1992)

13. Wlodaver et al. (FEBS Lett., 309, 59-64, 1992)

14. Ford et al, Protein Expression and Purification, 2, 95-107, 1991

15. Goosen et al, "Transformation and Gene Manipulation in Filamentous Fungi: an overview" in: Handbook of Applied Mycology, Vol. 4 (1992)

16. Romanos et al, Yeast 8:423-488 (1992)

17. EP-A-0,449,375

18. WO-A-98/04726

19. WO-A-98/30707

20. Alenkso and Clutterbuck, Fungal Genet. Biol 21: 373-397 (1997) 21. EP-A-0,635,574

22. WO-A-98/46772

23. WO-A-91/14772

**Claims**

1. A polypeptide, which is a β-glucanase obtainable from a fungus of the genus Talaromyces, such as the fungus Talaromyces emersonii, having the activity EC 3.2.1.4 (endoglucanase activity), comprising:

   (i) the amino acid sequence of SEQ ID No. 4; or
   (ii) a variant of (i) which is capable of cleaving β-D-glucan having at least 80 % identity over the whole length to the amino acid sequence of SEQ ID No. 4; or
   (iii) a fragment of (i) or (ii) which is capable of cleaving β-D-glucan.

2. The polypeptide according to Claim 1 wherein the variant (ii) has at least 85 % identity over the whole length to the amino acid sequence of SEQ ID No. 4 and/or the fragment of (iii) is at least 150 contiguous amino acids in length.

3. The polypeptide according to Claim 1 or 2, which has an optimum pH below 5.4 or an optimum temperature of >75 °C.

4. A polynucleotide comprising:

   (a) the nucleic acid sequence of SEQ ID No. 3 or a sequence encoding a polypeptide according to any preceding claim;
   (b) a sequence which is complementary to, or which hybridises to, a sequence as defined in (a) under stringent conditions;

(c) a fragment of at least 25 bp of SEQ ID No. 3;

(d) a sequence having at least 80%, identity to a sequence as defined in (a) or (b) ; or

(e) a sequence that is degenerate as a result of the genetic code to any one of the sequences as defined in (a) to (d)

(f) a modified sequence of Seq ID No. 3 with up to 100 nucleotide substitutions.

5. The polynucleotide according to Claim 4 wherein the sequence encodes a polypeptide having β-glucanase activity.

6. The polynucleotide according to any of Claims 4 to 5, which is a DNA sequence.

7. A vector comprising a polynucleotide sequence according to any one of Claims 4 to 6.

8. The vector according to Claim 7, which is an expression vector, such as where a DNA sequence according to Claim 6 is operably linked to a regulatory sequence.

9. A host cell, which comprises, as a heterologous sequence, a polynucleotide according to any of Claims 4 to 7.

10. A host cell, which expresses, as a heterologous protein, a polypeptide according to any of Claims 1 to 3.

11. A host cell transformed with the DNA sequence of Claim 6 as a heterologous sequence or a vector of Claim 8.

12. A process of producing a polypeptide according to any of Claims 1 to 3, the process comprising culturing a host cell as defined in any of Claims 9 to 11 under conditions that provide for expression of the polypeptide.

13. The use of a polypeptide according to any of Claims 1 to 3 for the manufacture of a medicament for treating hyperlipemia and/or high serum cholesterol and triglyceride levels in an individual or a disorder resulting there from.

14. A method of identifying a compound that modulates β-glucanase activity, the method comprising contacting a polypeptide according to any of Claims 1 or 3 with a test compound and monitoring for β-glucanase activity.

15. A method of treating plant material, the method comprising contacting the plant material with a polypeptide according to any one of Claims 1 to 3.

16. A method according to Claim 15 wherein the treatment comprises degrading or modifying cellulose, such as β-glucan, in the plant material.

17. A method according to Claim 16 for degrading or modifying plant cell walls.

18. A method according to any of Claims 15 to 17 wherein the treatment comprises cleaving of glucose subunits of a β-D-glucan component of the material.

19. A method according to any of Claims 15 to 17 wherein the material comprises a plant, plant pulp, plant extract or an edible foodstuff or ingredient therefore, or a fabric, textile or clothes containing plant material.

20. A method for reducing the viscosity of a plant material, the method comprising contacting the plant material with a polypeptide according to any one of Claims 1 to 3 in an amount and under conditions effective to degrade cellulose contained in the material.

21. Use of a polypeptide according to any one of Claims 1 to 3 in a method of treating plant material.

22. Use according to Claim 21 wherein the treatment comprises cleaving β-D-glucan polymers in the plant material.

23. Use of a polypeptide according to any one of claims 1 to 3 in a method of processing plant pulp, juice or extract which method comprises incubating the pulp, juice or extract with the polypeptide or composition to at least partially degrade cellulose.

24. Use of a polypeptide according to any of Claims 1 to 3 In brewing, distilling, biomethanation, dental hygiene, leather-treatment, paper manufacture, textile treatment or manufacture, baking or bread making, washing or detergent treatment, treating flower bulbs or In an animal feed.

**25.** A food or foodstuff comprising a polypeptide according to any of Claims 1 to 3.

**26.** A food or foodstuff according to Claim 25, which is an alcoholic beverage, bread, dough or tea.

**27.** A transgenic plant organism comprising a cell according to Claim 10 or 11.

**Patentansprüche**

**1.** Polypeptid, bei dem es sich um eine ß-Glucanase handelt, erhältlich aus einem Pilz der Gattung Talaromyces, wie dem Pilz Talaromyces emersonii, mit der Aktivität EC 3.2.1.4 (Endoglucanase-Aktivität), umfassend:

(i) die Aminosäuresequenz der SEQ ID NR: 4 oder
(ii) eine Variante von (i), die ß-D-Glucan spalten kann und mindestens 80% Identität über die gesamte Länge mit der Aminosäuresequenz der SEQ ID NR: 4 hat; oder
(iii) ein Fragment von (i) oder (ii), das ß-D-Glucan spalten kann.

**2.** Polypeptid nach Anspruch 1, wobei die Variante (ii) mindestens 85% Identität über die gesamte Länge mit der Aminosäuresequenz der SEQ ID NR: 4 hat und/oder das Fragment von (iii) mindestens 150 angrenzende Aminosäuren lang ist.

**3.** Polypeptid nach Anspruch 1 oder 2, das einen optimalen pH-Wert unter 5,4 oder eine optimale Temperatur von >75°C aufweist.

**4.** Polynukleotid, umfassend:

(a) die Nukleinsäuresequenz der SEQ ID NR: 3 oder eine Sequenz, die ein Polypeptid nach einem vorhergehenden Anspruch codiert;
(b) eine Sequenz, die zu einer Sequenz, wie in (a) definiert, komplementär ist oder unter stringenten Bedingungen daran hybridisiert;
(c) ein Fragment von mindestens 25 bp der SEQ ID NR: 3;
(d) eine Sequenz mit mindestens 80% Identität zu einer Sequenz, wie in (a) oder (b) definiert; oder
(e) eine Sequenz, die infolge des genetischen Codes zu einer der Sequenzen, wie in (a) bis (d) definiert, degeneriert ist
(f) eine modifizierte Sequenz von SEQ ID NR: 3 mit bis zu 100 Nukleotidsubstitutionen.

**5.** Polynukleotid nach Anspruch 4, wobei die Sequenz ein Polypeptid mit ß-Glucanase-Aktivität codiert.

**6.** Polynukleotid nach einem der Ansprüche 4 bis 5, das eine DNA-Sequenz ist.

**7.** Vektor, der eine Polynukleotidsequenz nach einem der Ansprüche 4 bis 6 umfasst.

**8.** Vektor nach Anspruch 7, bei dem es sich um einen Expressionsvektor handelt, beispielsweise in dem eine DNA-Sequenz nach Anspruch 6 mit einer regulatorischen Sequenz operativ verbunden ist.

**9.** Wirtszelle, die als heterologe Sequenz ein Polynukleotid nach einem der Ansprüche 4 bis 7 umfasst.

**10.** Wirtszelle, die als heterologes Protein ein Polypeptid nach einem der Ansprüche 1 bis 3 exprimiert.

**11.** Wirtszelle, die mit der DNA-Sequenz nach Anspruch 6 als heterologer Sequenz oder einem Vektor nach Anspruch 8 transformiert ist.

**12.** Verfahren zur Herstellung eines Polypeptids nach einem der Ansprüche 1 bis 3, umfassend das Kultivieren einer Wirtszelle, wie in einem der Ansprüche 9 bis 11 definiert, unter Bedingungen, welche die Expression des Polypeptids ergeben.

13. Verwendung eines Polypeptids nach einem der Ansprüche 1 bis 3 zur Herstellung eines Medikaments zur Behandlung von Hyperlipämie und/oder hohen Serum-Cholesterin- und -Triglyceridspiegeln in einem Individuum oder einer daraus resultierenden Erkrankung.

14. Verfahren zur Identifikation einer Verbindung, welche die ß-Glucanase-Aktivität moduliert, wobei das Verfahren das Zusammenbringen eines Polypeptids nach einem der Ansprüche 1 oder 3 mit einer Testverbindung und das Überwachen hinsichtlich ß-Glucanase-Aktivität umfasst.

15. Verfahren zur Behandlung von Pflanzenmaterial, umfassend das Zusammenbringen des Pflanzenmaterials mit einem Polypeptid nach einem der Ansprüche 1 bis 3.

16. Verfahren nach Anspruch 15, wobei die Behandlung den Abbau oder die Modifikation von Cellulose, wie β-Glucan, in dem Pflanzenmaterial umfasst.

17. Verfahren nach Anspruch 16 zum Abbau oder zur Modifikation von Pflanzenzellwänden.

18. Verfahren nach einem der Ansprüche 15 bis 17, wobei die Behandlung das Spalten von Glucose-Untereinheiten von einer ß-D-Glucan-Komponente des Materials umfasst.

19. Verfahren nach einem der Ansprüche 15 bis 17, wobei das Material eine Pflanze, Pflanzenbrei, Pflanzenextrakt oder ein essbares Nahrungsmittel oder einen Inhaltsstoff dafür oder einen Stoff, eine Textilie oder Bekleidung umfasst, der/die Pflanzenmaterial enthält.

20. Verfahren zur Verringerung der Viskosität eines Pflanzenmaterials, umfassend das Zusammenbringen des Pflanzenmaterials mit einem Polypeptid nach einem der Ansprüche 1 bis 3 in einer Menge und unter Bedingungen, welche den Abbau der in dem Material enthaltenen Cellulose bewirken.

21. Verwendung eines Polypeptids nach einem der Ansprüche 1 bis 3 in einem Verfahren zur Behandlung von Pflanzenmaterial.

22. Verwendung nach Anspruch 21, wobei die Behandlung das Spalten von ß-D-Glucan-Polymeren in dem Pflanzenmaterial umfasst.

23. Verwendung eines Polypeptids nach einem der Ansprüche 1 bis 3 in einem Verfahren zur Verarbeitung von Pflanzenbrei, -saft oder -extrakt, welches das Inkubieren des Breis, Saftes oder Extraktes mit dem Polypeptid oder der Zusammensetzung umfasst, um Cellulose zumindest partiell abzubauen.

24. Verwendung eines Polypeptids nach einem der Ansprüche 1 bis 3 zum Brauen, Destillieren, zur Biomethanation, Zahnhygiene, Lederbehandlung, Papierherstellung, Textilienbehandlung oder -herstellung, zum Backen oder Brotherstellen, zum Waschen oder zur Detergensbehandlung, zur Behandlung von Blumenzwiebeln oder in einem Tierfutter.

25. Lebens- oder Nahrungsmittel, umfassend ein Polypeptid nach einem der Ansprüche 1 bis 3.

26. Lebens- oder Nahrungsmittel nach Anspruch 25, bei dem es sich um ein alkoholisches Getränk, ein Brot, einen Teig oder Tee handelt.

27. Transgener Pflanzenorganismus, umfassend eine Zelle nach Anspruch 10 oder 11.

**Revendications**

1. Polypeptide qui est une β-glucanase que l'on peut obtenir à partir d'un champignon du genre *Talaromyces,* tel que le champignon *Talaromyces emersonii,* présentant l'activité EC 3.2.1.4 (activité endoglucanase), comportant :

   (i) la séquence d'acides aminés SEQ ID N° : 4 ; ou
   (ii) une variante de (i) susceptible de séparer le β-D-glucane, et ayant au moins 80 % d'identité sur la longueur totale par rapport à la séquence d'acides aminés de SEQ ID N° : 4 ; ou

(iii) un fragment de (i) ou de (ii) susceptible de séparer le β-D-glucane.

2. Polypeptide selon la revendication 1, dans lequel la variante (ii) a au moins 85 % d'identité sur la longueur totale par rapport à la séquence d'acides aminés de SEQ ID N° : 4 et/ou le fragment de (iii) a une longueur d'au moins 150 acides aminés contigus.

3. Polypeptide selon la revendication 1 ou 2, présentant un pH optimal inférieur à 5,4 ou une température optimale de >75 °C.

4. Polynucléotide comportant :

(a) la séquence d'acides nucléiques de SEQ ID N° : 3 ou une séquence codant pour un polypeptide selon l'une quelconque des revendications précédentes ;
(b) une séquence qui est complémentaire à, ou qui s'hybride à une séquence telle que définie dans (a) dans des conditions hautement astringentes ;
(c) un fragment d'au moins 25 pb de SEQ ID N° : 3 ;
(d) une séquence ayant au moins 80 % d'identité par rapport à une séquence telle que définie dans (a) ou (b) ; ou
(e) une séquence qui est dégénérée en raison de son code génétique par rapport à l'une quelconque des séquences telles que définies dans (a) à (d) ;
(f) une séquence modifiée de SEQ ID N° : 3 ayant jusqu'à 100 substitutions nucléotidiques.

5. Polynucléotide selon la revendication 4, dans lequel la séquence code pour un polypeptide ayant une activité β-glucanase.

6. Polynucléotide selon la revendication 4 ou 5, qui est une séquence ADN.

7. Vecteur comportant une séquence polynucléotidique selon l'une quelconque des revendications 4 à 6.

8. Vecteur selon la revendication 7, qui est un vecteur d'expression où une séquence ADN selon la revendication 6 est liée opérationnellement à une séquence régulatrice.

9. Cellule hôte, qui comporte un polynucléotide selon l'une quelconque des revendications 4 à 7 en tant que séquence hétérologue.

10. Cellule hôte, qui exprime un polypeptide selon l'une quelconque des revendications 1 à 3 en tant que protéine hétérologue.

11. Cellule hôte transformée avec la séquence ADN de la revendication 6 en tant que séquence hétérologue ou un vecteur de la revendication 8.

12. Procédé pour produire un polypeptide selon l'une quelconque des revendications 1 à 3, le procédé consistant à cultiver une cellule hôte telle que définie dans l'une quelconque des revendications 9 à 11 dans des conditions qui soient favorables à l'expression du polypeptide.

13. Utilisation d'un polypeptide selon l'une quelconque des revendications 1 à 3 pour fabriquer un médicament pour le traitement de l'hyperlipidémie et/ou de niveaux élevés de cholestérol sérique et de triglycérides chez un individu ou d'un trouble résultant de ceux-ci.

14. Procédé pour identifier un composé qui module l'activité β-glucanase, le procédé comportant les étapes consistant à mettre en contact un polypeptide selon l'une quelconque des revendications 1 ou 3 avec un composé de test et surveiller l'activité β-glucanase.

15. Procédé de traitement de matériel végétal, le procédé comportant la mise en contact du matériel végétal avec un polypeptide selon l'une quelconque des revendications 1 à 3.

16. Procédé selon la revendication 15, dans lequel le traitement comporte la dégradation ou la modification de cellulose, telle que du β-glucane, dans le matériel végétal.

**17.** Procédé selon la revendication 16, pour dégrader ou modifier des parois cellulaires végétales.

**18.** Procédé selon l'une quelconque des revendications 15 à 17, dans lequel le traitement comporte une séparation des sous-unités glucose d'un composant β-D-glucane du matériel.

**19.** Procédé selon l'une quelconque des revendications 15 à 17, dans lequel le matériel inclut un végétal, de la pulpe végétale, de l'extrait végétal ou un aliment comestible ou un ingrédient de celui-ci, ou un tissu, un textile ou des vêtements contenant du matériel végétal.

**20.** Procédé pour réduire la viscosité d'un matériel végétal, le procédé comportant la mise en contact du matériel végétal avec un polypeptide selon l'une quelconque des revendications 1 à 3 en une quantité et dans des conditions efficaces pour dégrader la cellulose contenue dans le matériel.

**21.** Utilisation d'un polypeptide selon l'une quelconque des revendications 1 à 3 dans un procédé de traitement de matériel végétal.

**22.** Utilisation selon la revendication 21, dans laquelle le traitement comporte la séparation de polymères de β-D-glucane dans le matériel végétal.

**23.** Utilisation d'un polypeptide selon l'une quelconque des revendications 1 à 3 dans un procédé de transformation de pulpe, de jus ou d'extrait végétal, ce procédé comportant l'incubation de la pulpe, du jus ou de l'extrait avec le polypeptide ou la composition pour dégrader au moins partiellement la cellulose.

**24.** Utilisation d'un polypeptide selon l'une quelconque des revendications 1 à 3 pour la brasserie, la distillation, la production de biométhane, l'hygiène dentaire, le traitement du cuir, la fabrication de papier, le traitement ou la fabrication de textile, la pâtisserie ou la boulangerie, le traitement nettoyant ou détergent, le traitement des bulbes à fleur ou dans une alimentation animale.

**25.** Nourriture ou aliment comportant un polypeptide selon l'une quelconque des revendications 1 à 3.

**26.** Nourriture ou aliment selon la revendication 25, qui est une boisson alcoolisée, du pain, de la pâte ou du thé.

**27.** Organisme végétal transgénique comportant une cellule selon la revendication 10 ou 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 184438 A
- EP 284603 A
- EP 134048 A
- EP 253455 A
- EP 096430 A
- EP 301670 A
- WO 9932617 A
- EP 0463706 A

- EP 0449375 A
- WO 9804726 A
- WO 9830707 A
- EP 0635574 A
- WO 9846772 A
- WO 9114772 A
- GB 00302263 A

**Non-patent literature cited in the description**

- **MOLONEY A. P. et al.** *BIOCHEMICAL JOURNAL,* 1985, vol. 225 (2), 365-374
- **Raper ; Fennell.** The Genus Aspergillus. The Williams & Wilkins Company, 1965, 293-344
- **Lever, M. ; Powell, J.C. ; Killip, M. ; Small, C.W.** *J. Lab. Clin. Med.,* 1973, vol. 82, 649-655
- **Sambrook et al.** Molecular Cloning: A laboratory manual. Cold Spring Harbor Laboratories, 1989
- **Innis et al.** PCR protocols, a guide to methods and applications. Academic Press, 1990
- **van Zeijl, C. et al.** *J. of BiotechnoL,* 1998, vol. 59, 221-224
- **Devereux et al.** *Nucleic Acids Research,* 1984, vol. 12, 387-395
- **Altschul S. F.** *J Mol Evol,* 1993, vol. 36, 290-300
- **Altschul, S, F et al.** *J Mol Biol,* 1990, vol. 215, 403-10

- **Henikoff ; Henikoff.** *Proc. Natl. Acad Sci. USA,* 1992, vol. 89, 10915-10919
- **Karlin ; Altschul.** *Proc. Natl. Acad Sci. USA,* 1993, vol. 90, 5873-5787
- **Cunningham ; Wells.** *Science,* 1989, vol. 244, 1081-1085
- **de Vos et al.** *Science,* 1992, vol. 255, 306-312
- **Smith et al.** *J. Mol. Biol.,* 1992, vol. 224, 899-904
- **Wlodaver et al.** *FEBS Lett.,* 1992, vol. 309, 59-64
- **Ford et al.** *Protein Expression and Purification,* 1991, vol. 2, 95-107
- Transformation and Gene Manipulation in Filamentous Fungi: an overview. **Goosen et al.** Handbook of Applied Mycology. 1992, vol. 4
- **Romanos et al.** *Yeast,* 1992, vol. 8, 423-488
- **Alenkso ; Clutterbuck.** *Fungal Genet. Biol,* 1997, vol. 21, 373-397